# EUROPEAN PATENT APPLICATION

(11) **EP 1 728 857 A2**
(43) Date of publication of application: **06.12.2006**
(21) Application number: 06120178.6
(22) Date of filing: 19.01.2001
(51) Int. Cl.: C12N 5/06, A61K 35/12, A61K 35/407, G01N 33/50

(54) **Liver tissue source**

(30) Priority: 19.01.2000 US 176798 P
(62) Divisional of application: 01903136.8
(71) Applicant: THE UNIVERSITY OF NORTH CAROLINA AT CHAPEL HILL, Chapel Hill, NC 27599-4105 (US)
(72) Inventor: Reid, Lola M., Chapel Hill, NC NC 27514 (US); Lecluyse, Edward L., Dept. of Drug & Disposition, Chapel Hill, NC 27599-7038 (US)
(74) Representative: van Westenbrugge, Andries

(57) **Abstract**

The instant invention provides, for the first time, the use of cadaveric organs from donors with non-beating hearts as a source of functional cells such as progenitor or stem cells for various medical purposes. More specifically, a method is disclosed whereby a tissue source of progenitor cells is obtained comprising harvesting tissue from a donor, wherein the donor has a non-beating heart for as long as about thirty hours postmortem and processing the cadaveric tissue to provide progenitor cells. The instant progenitors are used for various medical purposes as means of cell therapy, gene therapy, artificial organs, bioreactors, organ regeneration and the like.

## Description

### 1. Field of the Invention

This invention generally relates to procurement of diploid cells, including progenitor or stem cells, from tissues of donor cadavers with non-beating hearts.

### 2. Background of the Invention

There is a strong clinical and commercial interest in isolating and identifying immature progenitor cells from liver because of the impact that such a cell population could have in treating liver diseases. Each year in the United States, there are about 300,000 annual hospitalizations for liver failure. Liver transplants are curative for some forms of liver failure, and approximately 4800 transplants are performed a year in the United States. One of the limiting factors in liver transplantation is the availability of donor livers especially given the constraint that donor livers for organ transplantation must originate from patients having undergone brain death but not heart arrest. Livers from cadaveric (asystolic) donors have not been successful, although recent efforts to use such donors have supported the possibility of using them if the liver is obtained within a half hour of death.

Cell transplantation into the liver is an attractive alternative therapy for most liver diseases. The surgical procedures for cell transplantation are minor relative to those needed for whole organ transplantation and, therefore, can be used in patients with various surgical risks such as age or infirmity. The use of human liver cells is superior to liver cells derived from other mammals because the potential pathogens, if any, are of human origin and could be both better tolerated by patients and easily screened before use.

Attempts have been made in the past to obtain the hepatic progenitor cell population, suggested to be the most versatile population for cell and gene therapy of the liver. U.S. Pat. Nos. 5,576,207 and 5,789,246 to Reid et al. who utilized cell surface markers and side scatter flow cytometry to provide a defined subpopulation in the liver. Hepatic progenitors are diploid cells that themselves or their progeny are capable of differentiating into hepatocytes.

Liver progenitors are also extremely useful for production of growth factors. These could be associated with their own growth or that of other progenitors in the liver (e.g. hemopoietic or mesenchymal progenitors) and could also include as yet undiscovered growth factors associated with early steps in the dedication of hepatic progenitor cells to a particular lineage. These novel growth factors could have potential in treating liver disease or in controlling liver cancers, now recognized to be transformants of the liver progenitors.

Furthermore, liver progenitors are vehicles for gene therapy, wherein the inserted genetically transformed or normal hepatic progenitors promote the health of the individual into whom such hepatic progenitors are transplanted.

Attempts to perform liver cell transplantation have made use of unfractionated mature liver cells and have shown some measure of efficacy. However, the successes require injection of large numbers of cells (10-20 billion), since the cells have limited growth potential *in vivo.* Furthermore, the introduction of substantial numbers of large mature liver cells (average cell diameter 25-50 µm) is complicated by their tendency to form large aggregates upon injection, resulting in potentially fatal emboli. Moreover, these cells elicit a marked immunological rejection response forcing patients to be maintained on immunosuppressive drugs for the remainder of their lives.

Mature, differentiated liver cells are distinguishable from progenitor liver cells by several criteria. The differentiated cells tend to form clumps or aggregates, which, if injected into a patient, result in a risk of emboli formation. The differentiated cells are peculiarly resistant to cryopreservation and are notably immunogenic. Moreover, as the replicative capacity of the differentiated cells is limited, transplantation with differentiated cells has few, if any advantages compared to organ transplantation, and disadvantages that include a more elaborate preparation procedure.

The shortage of essential organs, e.g., heart, liver, pancreas, lung, and kidney, for transplantation or other medical purposes which require donor tissues is due to the limited availability of organs that are still functional. Currently, the organs intended for transplantation are retrieved from brain-dead donors whose hearts are still beating. If the heart stops, the blood circulation is arrested (ischemia), which interrupts the oxygenation of tissues (anoxia) and consequently, organs are damaged ischemically within a very short period of time resulting in almost certain probability that such organs will not function when transplanted. In general, no organs are used after heart arrest and, experimentally, none are used after more than one-half hour from the time of heart arrest or asystole. Currently, only 1-2% of deaths in hospitals meets the brain-death-heart-beating criteria. However, a large and yet untapped source of organs for transplantation is available, many from accident victims who either die at the site of an injury or have a short post-trauma survival time. These accident victims are not used as organ donors because of the ischemic damage. Organs such as liver, brain and heart are among the most ischemia-sensitive tissues. For example, anoxic and ischemic brain injuries from cardiac arrest result in damage to the brain and associated neurologic tissues after about four minutes. The heart can survive intact up to four hours after cardiac arrest. The liver can functionally survive functionally for no longer than one hour and transplants from non-heart-beating donors (NHBDs) are recommended to be carried out preferably within the first thirty-five minutes of exposure to warm ischemia (see the abstracts, incorporated by reference, of the articles by Ong HS, Soo KC, Joseph VT, Tan SY, Jeyaraj PR. The viability of liver grafts for transplantation after prolonged warm ischemia. Ann Acad Med Singapore 1999 Jan;28(1):25-30; Hong HQ, Yin HR, Zhu SL, Lin YT. The results of transplant livers from selected non-heart-beating cadaver donors. Hiroshima J Med Sci 1991 Sep;40(3):87-91). Under present medical regulations, the time prior to that which a potentially transferable organ can be salvaged is usually delayed. This occurs because the potential donor must first be brought to a hospital or to a morgue. The family must then sign organ donation forms. Only after the organ donation procedures are complete, a surgical team is permitted access to the body to harvest the organs. Because of the elapsed time due to these procedures on many occasions the organs are already irreversibly damaged or are no longer viable. Accordingly the prior art provides a large number of methods and processes for protecting donor organs from ischemic damage. See for example U.S. Pat. Nos. 5,702,881; 5,660,976; 5,752,929; 5,863,296; 5,855,617; 5,843,024; 5,827,222; 5,723,282; 5,514,536; and 4,723,939 among many others and incorporated herein by way of reference. Despite the abundance of prior art references directed at means of protecting donor organs from losing functionality, the prior art is silent when it comes to the use of cadavers whose hearts were arrested beyond the irreversible time point. Only three U.S. Pat. Nos. (5,843,024; 5,702,881; 4,723,939) seem to deal with non-beating-heart donors. This prior art fails to teach the use "irreparable" organs for isolating progenitor cells from them. While methods of isolating liver precursor cells are known in the art (see, for example U.S. Pat. Nos. 5,576,207 and 5,789,246, incorporated herein by reference) until the reduction to the practice of the present invention it was not known that precursor hepatic cells can be isolated from what was considered in the prior art as a "useless" organ.

Technologies developed from the advances in the understanding of human liver progenitor cells and their isolation and expansion, as pioneered by the inventors of the invention described herein, offer a major impact on the morbidity and mortality associated with liver disease by offering a novel cell population which is extremely useful for cell transplantation into the liver.

Accordingly, there is a long-felt need for effective means of using organs from cadavers with arrested blood circulation or non-beating heart as a source of organs or organ-equivalents for medical purposes.

### 3. Summary of the Invention

The present invention is directed to a method of providing a tissue having at least one diploid cell population as a source of diploid cells. The method comprises (a) harvesting a tissue from a donor having a non-beating heart at a time when the tissue is harvested, the tissue harvested being suspected of having at least one diploid cell population; (b) processing the harvested tissue to obtain a population of cells substantially enriched in diploid cells. Preferably, the donor is not a fetus and is selected from a neonate, an infant, a child, a juvenile, or an adult. The diploid cells obtained from the present method include progenitors.

In a particular embodiment of the invention, the processing step comprises processing the harvested tissue to provide a substantially single cell suspension. This single cell suspension can be processed further by separating the substantially single cell suspension into two or more fractions, typically, three or more, preferably, four or more. In this separating step the larger cells are separated from the smaller cells, higher density cells from lower density cells, or both. Any method known to those of ordinary skill in the art of separating the cells into fractions can be used. A convenient method is centrifugation, first at slower speeds, then increasingly faster speeds. The fractions consisting essentially of smaller cells, lower density cells, or both, are further processed to provide a population of cells substantially enriched in diploid cells. In particular, examples of diploid cells, which are desirable, include progenitors that express alpha-fetoprotein, particularly, liver progenitors.

The preferred tissues of the present invention are those which have been harvested within about six hours after the donor's heartbeat ceased, preferably, within about three hours after the heartbeat ceased, more preferably, within about two hours after the heartbeat ceased and, most preferably, within about one hour after the heartbeat ceased. The sooner the tissue is harvested after the donor's heartbeat ceased the better, however. Hence, still more preferred, are tissues harvested within about 45, 30, or 15 minutes after the donor's heartbeat ceased. A variety of tissues can be harvested and processed to obtain diploid cells, including adrenal gland, blood vessel, bone marrow, cornea, retina, islets of Langerhans, bile duct, lens, lung, kidney, heart, gut, ovary, pancreas, prostate, parathyroid, pineal, pituitary, skin, testis, bladder, brain, spinal cord, spleen, thymus, thyroid, or liver.

The present invention is also directed to a composition comprising a population of cells substantially enriched in diploid cells, especially those that express alpha-fetoprotein, obtained by the method of the invention.

The present invention provides a significant breakthrough in the field of acquisition of donor organs and tissues and provides means of obtaining a tissue source of progenitor cells and diploid adult cells. This invention was completely unexpected, since all known prior art references regarded ischemically damaged organs as being totally useless for any meaningful purpose. The preferred means comprise harvesting tissue from a donor, wherein the donor has a non-beating heart and processing the tissue to provide diploid cells that can include progenitor or stem cells.

Preferably this invention comprises a method of providing a tissue source of liver diploid cells including progenitor cells, which comprises harvesting liver tissue from a donor, wherein the donor has a non-beating heart and processing the tissue to provide diploid cells and/or hepatic progenitor cells. Such cells are useful for example in repopulating damaged liver parenchyma or reconstituting liver in a host in need thereof. While any animal donor is equally suitable, the preferred donor is a human. Animals such as pigs and primates are equally suitable.

Accordingly, it is an object of this invention to obtain such organs or tissues within about twenty four hours or more after the heartbeat ceased. Even though the time limitation is not binding it is preferable that the tissue is obtained within about sixteen hours after the heartbeat ceased. More preferably the tissue is obtained within about ten hours after the heartbeat ceased. Yet more preferably the tissue is obtained within about six hours after the heartbeat ceased. Even more preferable the tissue is obtained within about three hours after the heartbeat ceased. Another preferred time period is when the tissue is obtained is within about one hour after the heartbeat ceased. Regardless of the time period the diploid cells and progenitors are resistant to ischemia. Harvested tissues are either perfused with suitable perfusion media or not perfused for further processing.

While the tissue is preferably cooled to about room temperature it is equally advantageous to have the tissue cooled to about 4 °C. The tissue can be cooled for all or part of the ischemic time. That is, the organ can be subjected to a combination of warm and cold ischemia.

Within the scope of the invention it is preferable that the donor is a neonate, an infant, a child, a juvenile, or an adult. Fetal tissues deemed unsuitable due to the presumed ischemia are also contemplated within the scope of this invention. While the age of the donor is not critical, it is desirable that the donor is between about 0 years and about 77 years old, more preferably less than about 50 years old.

The preferred tissues useful in this invention comprise adrenal gland, blood vessel, bone marrow, cornea, islets of Langerhans, lens, liver, ovary, pancreas, parathyroid, pineal, pituitary, skin, testis, thymus, thyroid or combinations thereof. Preferably the tissue is liver.

Another embodiment of the present invention is to provide processing means which result in a substantially single cell suspension from such tissues. Preferred processing methods additionally comprise a debulking step, which substantially reduces the number of polyploid or mature cells in the suspension, to provide a debulked suspension enriched in diploid cells and/or progenitors exhibiting at least one marker associated with at least one cell lineage. Without limiting to such means the processing steps include separating cells by size or density.

Preferably the processing additionally comprises selecting from the suspension those cells that express at least one marker associated with at least one cell lineage, whereby the at least one cell lineage includes at least one of hepatic, hematopoietic, or mesenchymal cell lineage. It is a further object of this invention to provide diploid cells and/or progenitor cells having the capacity to develop into hepatocytes, biliary cells, or a combination thereof.

It is preferable that donor cells of the invention express at least one marker including alpha-fetoprotein, albumin, bone sialoprotein, CD14, CD34, CD38, CD90, CD45, CD117, ICAM-1, collagen type I, collagen type II, collagen type III, glycophorin A, or osteopontin, either alone or in advantageous combination.

As a further object of the invention a method of therapy is provided, in which progenitor cells are used as a cellular transplant, a bioreactor, an artificial organ, etc. The preferred medical conditions and needs comprise Crigler-Najjar syndrome, tyrosinemia, cirrhosis, acute liver failure, diabetes, and other liver and liver-related conditions known m the art. In general, patients are treated who may suffer from at least one liver disorder selected from the group consisting of inflammation of the liver, viral hepatitis, toxic liver cell damage, fibrosis of the liver, cirrhosis of the liver, liver congestion, liver dystrophy, fatty degeneration of liver cells, fatty liver, disturbances of the detoxification function, disturbances of the excretory function of the liver, disturbances of the conjugational function of the liver, disturbances of the synthesizing function of the liver portal hypertension due to a liver disease, or a liver failure coma, and intoxication by protein degradation products or ammonia. These malfunctions result in diseases such as Alagille syndrome, alcoholic liver disease, alpha-1-antitrypsin deficiency, autoimmune hepatitis, biliary atresia, biliary ductopenia, bone marrow failure, Budd-Chiari syndrome, Byler disease, Crigler-Najjar syndrome, Caroli disease, cholestatic pruritus, cholelithiasis, conjugated hyperbilirubinemia, chronic graft-versus-host disease, cryptogenic liver disease, diabetes, Dubin-Johnson syndrome, erythrohepatic protoporphyria, extrahepatic bile duct carcinoma, familial hypercholesterolemia, galactosemia, Gilbert syndrome, glycogen storage disease, hemangioma, hemochromatosis, hepatic encephalopathy, hepatocholangitis, hepatomalacia, hepatomegalia, hepatocarcinoma, hepatoblastoma, hereditary hemochromatosis, jaundice, intrahepatic cholestasis, liver cysts, liver transplantation, liver failure associated with Bacillus cereus, mixed cryoglobulinemia, ornithine transcarbamylase deficiency, peliosis hepatis, porphyria cutanea tarda, primary biliary cirrhosis, refractory ascites, Rotor syndrome, sarcoidosis, sclerosing cholangitis, steatosis, Summerskill syndrome, thrombocytopenia, tyrosinemia, variceal bleeding, venocclusive disease of the liver, and Wilson disease among many others, and are advantageously treated with the methods and compositions of the instant invention.

Without limiting to above embodiments the methods of gene therapy are also contemplated, which comprise means well known in the art including but not limited to introduction of a vector into diploid and/or progenitor cells, then transplanting to a host in need thereof. Conditions and target genes can comprise the LDL receptor gene in familial hypercholesterolemia, the clotting factor genes for factors VIII and IX in hemophilia, the alpha-1-antitrypsin gene in emphysema, the phenylalanine hydroxylase gene in phenylketonuria, the ornithine transcarbamylase gene in hyperammonemia, and complement protein genes in various forms of complement deficiencies, and other medical conditions which will be advantageously treated or cured by means of gene therapy.

Other desired embodiments include genes encoding carbamoyl synthetase I, ornithine transcarbamylase, arginosuccinate synthetase, arginosuccinate lyase, arginase fumarylacetoacetate hydrolase, phenylalanine hydroxylase, alpha-1 antitrypsin, glucose-6-phosphatase, low-density-lipoprotein receptor, porphobilinogen deaminase, carbamoyl synthetase I, ornithine transcarbamylase, arginosuccinate synthetase, arginosuccinate lyase, arginase, factors VIII or IX, cystathione beta-synthase, branched chain ketoacid decarboxylase, albumin, isovaleryl-CoA dehydrogenase, propionyl CoA carboxylase, methyl malonyl CoA mutase, glutaryl CoA dehydrogenase, insulin, transferrin, beta-glucosidase, pyruvate carboxylase, hepatic phosphorylase, phosphorylase kinase, glycine decarboxylase, H-protein, T-protein, Menkes disease protein, or the product of Wilson's disease gene.

The present invention also relates to a method of isolation and cryopreservation of diploid cells and/or progenitors from human liver which includes (a) processing human liver tissue to provide a substantially single cell suspension including diploid adult cells, progenitors and non-progenitors of one or more cell lineages found in human liver; (b) subjecting the suspension to a debulking step, which reduces substantially the number of non-progenitors in the suspension, to provide a debulked suspension enriched in progenitors exhibiting one or more markers associated with at least one of the cell lineages; and (c) selecting from the debulked suspension those cells, which themselves, their progeny, or more mature forms thereof express one or more markers associated with several liver cell lineages; and (d) suspending the cells under conditions optimal for cryopreservation. More preferably liver progenitors expressing cytoplasmic proteins such as alpha-fetoprotein are selected. Processing or debulking steps of this invention preferably include a density gradient centrifugation of the liver cell suspension to separate the cells according to their buoyant density and size which are associated with one or more gradient fractions having a lower buoyant density.

Non-progenitors of the liver cell suspension includes mature hepatic, hemopoietic, and mesenchymal cells. Negative selection of the non-progenitors includes the use of markers associated with mature hepatic cells, such as connexin32, markers associated with hemopoietic cells, such as glycophorin A and CD45, or markers associated with mature mesenchymal cells, such as retinoids, or von Willebrand Factor.

A further aspect of this invention provides for liver cell progenitors of hepatic, hematopoietic, or mesenchymal origin. These cell lineages, their progenies or their more mature forms are selected by antigenic markers selected from the group consisting of CD14, CD34, CD38, CD45, CD117, ICAM, glycophorin A, and/or cytoplasmic markers such as alpha-fetoprotein-like immunoreactivity, albumin-like immunoreactivity, or both. Alpha-fetoprotein derives from variant forms of mRNA some of which are unique to hepatic progenitor cells and some to hemopoietic progenitor cells. The liver progenitors of this invention can be isolated from the liver of a fetus, a neonate, an infant, a child, a juvenile, or an adult.

In accordance with yet a further aspect of this invention, isolated human liver progenitors, a subpopulation of the diploid cells, are isolated in a highly enriched to substantially pure form. Such liver progenitors contain hepatic, hemopoietic and mesenchymal progenitors. The hepatic progenitors have the capacity to develop into hepatocytes, biliary cells, or a combination thereof; the hematopoietic progenitors have the capacity to develop into macrophages, neutrophils, granulocytes, lymphocytes, platelets, neutrophils eosinophils, basophils, or a combination thereof. The mesenchymal progenitors have the capacity to develop into endothelial cells, stromal cells, hepatic stellate cells (Ito cells), cartilage cells, bone cells or combinations thereof. The method of this invention can be used to select mesenchymal progenitors expressing CD34, osteopontin, bone sialoprotein, collagen types I, II, or III, or a combination thereof.

The present inventors overcome many of the above difficulties making diploid cells, including progenitor cells, ideal for use in cell and gene therapies and for bioartificial organs. The cells are small, therefore minimizing the formation of large emboli. Also, the cells have extensive growth potential meaning that fewer cells are needed for reconstitution of liver tissue in a patient. Finally, the progenitors have minimal antigenic markers that might elicit immunological rejection providing hope that little or no immunosuppressive drugs might be needed.

A further aspect of this invention provides for liver progenitors that harbor exogenous nucleic acid. Such exogenous nucleic acid can encode one or more polypeptides of interest, or can promote the expression of one or more polypeptides of interest.

In accordance with yet a further aspect of this invention, there is provided a method of alleviating the negative effects of one or more human disorders or dysfunctions by administering to an individual suffering from such negative effects an effective amount of isolated human diploid liver cells and/or progenitors. The progenitors can be administered parenterally via a vascular vessel, or administered directly into the liver. The direct administration can be effected surgically via portal vein, mesenteric vein, hepatic bile duct, or combinations thereof. Alternatively, the liver progenitors can be administered into an ectopic site of the individual, such as spleen.

The human disorders or dysfunctions that could be alleviated by the method of this invention include: hepatocholangitis, hepatomalacia, hepatomegalia, cirrhosis, fibrosis, hepatitis, acute liver failure, chronic liver failure, or inborn errors of metabolism, hepatocarcinoma, or hepatoblastoma. Cancer of the liver could be a primary site of cancer or one that has metastasized into the liver. The metastatic tumor could be derived from any number of primary sites including, intestine, prostate, breast, kidney, pancreas, skin, brain, lung or a combination thereof. The hepatic disease or dysfunction that can be treated with this methods also includes liver disease or dysfunction associated with an impairment in the mitochondrial compartment of hepatic tissues and can consist of chronic liver disease, fulminant hepatic failure, viral-induced liver disease, metabolic liver disease, and hepatic dysfunction associated with sepsis or liver trauma.

In accordance with yet a further aspect of the invention, a bioreactor is provided which includes (i) biological material comprising (a) isolated progenitors from human liver, their progeny, their maturing or differentiated descendants, or combinations thereof, (b) extracellular matrix, and (c) media; (ii) one or more compartments holding said biological material or the components comprising said biological material; and (iii) one or more connecting ports. The biological material of the bioreactor can optionally also include: (d) hormones, growth factors, or nutritional supplements, or (e) plasma, serum, lymph, or products derived therefrom.

The bioreactor is adapted for sustaining said progenitors in a viable, functional state, and can sustain liver progenitors for a period ranging from about one week or longer. Specifically, the bioreactor is adapted for use as an artificial liver, for product manufacturing, toxicological studies, or metabolic studies, including studies involving the activity of cytochrome P450, or other types of drug metabolism.

In accordance with yet another aspect of this invention, a composition of isolated human liver progenitors, or a suspension enriched in progenitors obtained from human liver is provided. The cell suspension is provided in a pharmaceutically acceptable carrier or diluent and is administered to a subject in need of treatment. The composition of this invention includes liver progenitors that exhibit one or more markers associated with at least one of one or more cell lineages found in human liver and are substantially free of mature cells. More particularly, isolated liver progenitors are derived from one or more liver cell lineages including hepatic, hematopoietic, or mesenchymal cell lineages and themselves, their progeny, or more mature forms of the progenitors thereof express at least one or more of antigenic markers CD14, CD34, CD38, CD90, or CD117, CD45, glycophorin A, and cytoplasmic markers of alpha fetoprotein-like immunoreactivity, albumin-like immunoreactivity, or both.

In accordance with yet another embodiment of this invention, a cell culture of liver progenitors is provided which includes isolated progenitors from human liver, their progeny, their maturing or differentiated descendants, or combinations thereof. The cell culture additionally includes extracellular matrix comprising one or more collagens, one or more adhesion proteins (laminins, fibronectins), and other components such as proteoglycans (such as heparan sulfate proteoglycans); or an individual matrix component. Matrix component includes fragments of matrix components; matrix mimetics that can be synthetic and/or biodegradable materials (i.e. microspheres) coated with one or more of the factors from one of the classes of extracellular matrices. The cell culture additionally includes basal media and other nutrients; hormones and/or growth factors, with or without a biological fluid such as serum, plasma or lymph. Additionally, the culture media could contain one or more compartments that holds the biological material such as a culture dish , flask, roller bottle, transwell or other such container.

The cultures or bioreactors of this invention could be used to produce various medically important cell-secreted factors including but not limited to enzymes, hormones, cytokines, antigens, antibodies, clotting factors, anti-sense RNA, regulatory proteins, ribozymes, fusion proteins and the like. The cultures are suitable to supply a therapeutic protein such as Factor VIII, Factor IX, Factor VII, erythropoietin, alpha-1-antitrypsin, calcitonin, growth hormone, insulin, low density lipoprotein, apolipoprotein E, IL-2 receptor and its antagonists, superoxide dismutase, immune response modifiers, parathyroid hormone, the interferons (IFN alpha, beta, or gamma), nerve growth factors, glucocerebrosidase, colony stimulating factor, interleukins (IL) 1 to 15, granulocyte colony stimulating factor (G-CSF), granulocyte, macrophage-colony stimulating factor (GM-CSF), macrophage-colony stimulating factor (M-CSF), fibroblast growth factor (FGF), platelet-derived growth factor (PDGF), adenosine deaminase, insulin-like growth factors (IGF-1 and IGF-2), megakaryocyte promoting ligand MPL, thrombopoietin, etc.

As a further embodiment of this invention a pharmaceutical composition is provided which is useful for treating and preventing a liver disease. The composition comprises an effective amount of cadaveric liver progenitor cells and a pharmaceutical carrier. The liver diseases of interest include acute or chronic liver disease of toxic, metabolic, genetic, and/or infective origin or of degenerative nature, or liver damage resulting from the use of drugs or substances injurious to the liver. Preferably among these conditions and diseases are inflammation of the liver, viral hepatitis, toxic liver cell damage, fibrosis of the liver, cirrhosis of the liver, liver congestion, liver dystrophy, fatty degeneration of liver cells, fatty liver, disturbances of the detoxification function, disturbances of the excretory function of the liver, disturbances of the conjugational function of the liver, disturbances of the synthesizing function of the liver portal hypertension due to a liver disease, or a liver failure coma, and intoxication by protein degradation products of ammonia. More specifically these include but are not limited to Alagille syndrome, alcoholic liver disease, alpha-1-antitrypsin deficiency, autoimmune hepatitis, biliary ductopenia, bone marrow failure, Budd-Chiari syndrome, biliary atresia, Byler disease, Crigler-Najjar syndrome, Caroli disease, cholestatic pruritus, cholelithiasis, conjugated hyperbilirubinemia, chronic graft-versus-host disease, cryptogenic liver disease, diabetes, Dubin-Johnson syndrome, erythrohepatic protoporphyria, extrahepatic bile duct carcinoma, familial hypercholesterolemia, galactosemia, Gilbert syndrome, glycogen storage disease, hemangioma, hemochromatosis, hepatic encephalopathy, hepatocholangitis, hepatomalacia, hepatomegalia, hepatocarcinoma, hepatoblastoma, hereditary hemochromatosis, jaundice, intrahepatic cholestasis, liver cysts, liver transplantation, liver failure associated with Bacillus cereus, mixed cryoglobulinemia, ornithine transcarbamylase deficiency, peliosis hepatis, porphyria cutanea tarda, primary biliary cirrhosis, refractory ascites, Rotor syndrome, sarcoidosis, sclerosing cholangitis, steatosis, Summerskill syndrome, thrombocytopenia, tyrosinanemia, variceal bleeding, venocclusive disease of the liver, Wilson disease and combinations thereof.

Other objects will be made known to the skilled artisan in view of the following detailed disclosure.

### 4. Brief Description of the Figures

Figures 1a and 1b illustrate the effect of warm ischemia on the proportion of isolated cells with small and large nuclei.
Figures 2a and 2b illustrate PCR analysis of truncated alpha-fetoprotein (AFP) in hemopoietic cells.
Figure 3 illustrates the relationship between storage time at -170°C and viability of thawed fetal liver cells.
Figures 4a and 4b illustrate typical univariate histograms of fetal liver cell suspensions analyzed by fluorescence activated cell sorting (FACS).
Figure 5 illustrates percent of cells expressing surface markers CD14, CD34, CD38, CD45 and Glycophorin A (GA) in unfractionated liver cell suspensions
Figure 6 illustrates percentage of cells in the original cell suspension expressing alpha-fetoprotein and other antigenic markers
Figures 7a, 7b and 7c illustrate alpha-fetoprotein expression before and after depletion of red blood cells.
Figures 8a, 8b, 8c, 8d, 8e and 8f illustrates FACS analysis of fetal liver cell suspension for co-expression of CD14, CD38 and AFP.
Figure 9 illustrates CD 14 and CD38 enrich for AFP-positive cells.
Figures 10a, 10b, 10c and 10d illustrate fluorescence microscopy of human hepatic progenitor cells.
Figures 11a, 11b, 11c and 11d illustrate representative cells selected by expression of AFP.
Figures 12a, 12b and 12c show that there are two AFP positive cells in this field.
Figures 13a and 13b illustrate cells that are labeled with calcein (A) to show all cell types.

### 5. Detailed Description of the Invention

In the description that follows, a number of terms are used extensively to describe the invention. In order to provide a clear and consistent understanding of the specification and claims, the following definitions are provided.

Alpha-fetoprotein-like immunoreactivity: Any immune reactions caused by alpha-fetoprotein. Alpha-fetoprotein derives from variant forms of mRNA some of which are unique to hepatic progenitor cells and some to hemopoietic progenitor cells.

Committed progenitors: Immature cells that have a single fate such as hepatocytic committed progenitors (giving rise to hepatocytes) or biliary committed progenitors (giving rise to bile ducts). The commitment process is not understood on a molecular level. Rather, it is recognized to have occurred only empirically when the fates of cells have narrowed from that of a predecessor.

Hepatic cells: A subpopulation of liver cells, which includes hepatocytes and biliary cells.

Liver cells: As used herein, the term "liver cells" refers to all type of cells present in normal liver, regardless of their origin or fate.

Stem cells: As used herein, the term "stem cells" refers to immature cells that can give rise to daughter cells with more than one fate. Some daughter cells are identical to the parent and some "commit" to a specific fate. Totipotent stem cells have self-renewal (self-maintaining) capacity, whereas determined stem cells have questionable self-renewal capacity. Stem cells can regenerate during a regenerative proliferative process.

Hepatic progenitors: These cells give rise to hepatocytes and biliary cells. The hepatic progenitors include three subpopulations: "hepatic stem cells", "committed hepatocytic progenitors", and "committed biliary progenitors," the last two being immature cells that are descendants of the hepatic stem cell and that have a single fate, either hepatocytes or biliary cells, but not both.

Hepatic stem cells: A subpopulation of hepatic progenitors.

Liver progenitors: A cell population from liver, including hepatic progenitors, hemopoietic progenitors and mesenchymal progenitors.

Oval cell: a small cell (<15 microns) with oval shaped nuclei proliferating in animals exposed to oncogenic insults. These cells are thought to derive from liver progenitors and are partially or completely transformed.

The "liver" is a large organ located in the most forward part of the abdomen, resting against the muscular partition between the abdominal and chest cavities. The liver is essential for life and performs over 100 important functions, such as detoxifying poisons and drugs, metabolizing fats, storing carbohydrates, manufacturing bile, plasma proteins and other substances, and assisting in blood clotting. Liver disease is often difficult to detect until the illness becomes severe because there is an overabundance of liver tissue, and the liver can partially regenerate itself. The signs of liver disease vary with the degree and location of damage. Various blood tests are necessary to discover the extent and the nature of liver damage.

The term "growth factor" as used herein refers to those factors required to regulate developmental events or required to regulate expression of genes encoding other secreted proteins that can participate in intercellular communication and coordination of development and includes, but is not limited to hepatocyte growth factor (HGF), insulin-like growth factor-I and II (IGF-I and II), epidermal growth factor (EGF), type a and type b transforming growth factor (TGF-alpha and TGF-beta), nerve growth factor (NGF), fibroblast growth factor (FGF), platelet-derived growth factor (PDGF), sarcoma growth factor (SGF), granulocyte macrophage colony stimulating growth factor (GM-CSF), vascular endothelial growth factor (VEGF), prolactin and growth hormone releasing factor (GHRF) and various hemopoietic growth factors such as interleukins (IL) IL-1, IL-2, IL-3, IL-4, IL-5, IL-6, IL-7, IL-8, IL-10, IL-11, etc., erythroid differentiation factor (HDF) or follicle-stimulating hormone releasing protein (FRP), inhibin, stem cell proliferation factor (SCPF) and active fragments, subunits, derivatives and combinations of these proteins among many others known in the art. Generally, as used hereinafter, the growth factor refers to a secreted protein which is selected from the group consisting of a cytokine, a lymphokine, an interleukin, a colony-stimulating factor, a hormone, a chemotactic factor, an anti-chemotactic factor, a coagulation factor, a thrombolytic protein, a complement protein, an enzyme, an immunoglobulin, and an antigen.

Hemopoiesis: Yielding blood cells with cell fates of lymphocytes (B and T), platelets, macrophages, neutrophils, and granulocytes.

Mesengenesis: Yielding mesenchymal derivatives with cell fates of endothelia, fat cells, stromal cells, cartilage, and even bone (the last two occurring in the liver only under disease conditions).

Cell Therapy: As used herein, the term "cell therapy" refers to the in vivo or ex vivo transfer of defined cell populations used as an autologous or allogenic material and transplanted to, or in the vicinity of, specific target cells of a patient. Cells can be transplanted in any suitable media, carrier or diluents, or any type of drug delivery systems including, microcarriers, beads, microsomes, microspheres, vesicles and so on.

Gene Therapy: As used herein, the term "gene therapy" refers to the in vivo or ex vivo transfer of defined genetic material to specific target cells for a patient in need thereof, thereby altering the genotype and, in most situations, altering the phenotype of those target cells for the ultimate purpose of preventing or altering a particular disease state. As this definition states, the underlying premise is that these therapeutic genetic procedures are designed to ultimately prevent, treat, or alter an overt or covert pathological condition. In most situations, the ultimate therapeutic goal of gene therapy procedures is to alter the phenotype of specific target cell population.

CD: "Cluster of differentiation" or "common determinant" as used herein refers to cell surface molecules recognized by monoclonal antibodies. Expression of some CDs are specific for cells of a particular lineage or maturational pathway, and the expression of others varies according to the state of activation, position, or differentiation of the same cells.
- Ploidy:: chromosome number within a cell.
- Diploid:: two sets of chromosomes per cell.
- Tetraploid:: four sets of chromosomes per cell.
- Octaploid:: eight sets of chromosomes per cell.
- Polyploid:: more than two sets of chromosomes per cell.

The cells of the normal fetal or neonatal liver are diploid. By the young adult stage, the liver is a mixture of diploid and polyploid cells. In rodents, the liver is about 90% polyploid and only about 10% diploid cells. In humans, the liver of young adults is composed of 50% diploid and 50% polyploid cells.

Without limiting to liver, other progenitor cells from various cadaveric tissues are disclosed and claimed by this invention. As used hereinafter the term "cadaveric tissue" does not include tissue from dead fetuses obtained by means such as premature termination of pregnancy by a surgical procedure. Humans delivered by natural or assisted birth are considered as neonates or infants but not as fetuses. Accordingly the age of a human starts at "0" at the time of birth or delivery and not from the time of conception. Thus a neonate dead at the time of birth will be considered as a cadaver and not as a fetus. Freshly obtained fetal tissues have been used as a source of some progenitor cells and as such they are excluded from the breadth of claims of this invention. However, fetal tissue which is considered unsuitable for further medical use due to the presumed ischemia effect is still suitable for the purposes of this invention.

When the terms "one," "a," or "an" are used in this disclosure, they mean "at least one" or "one or more," unless otherwise indicated.

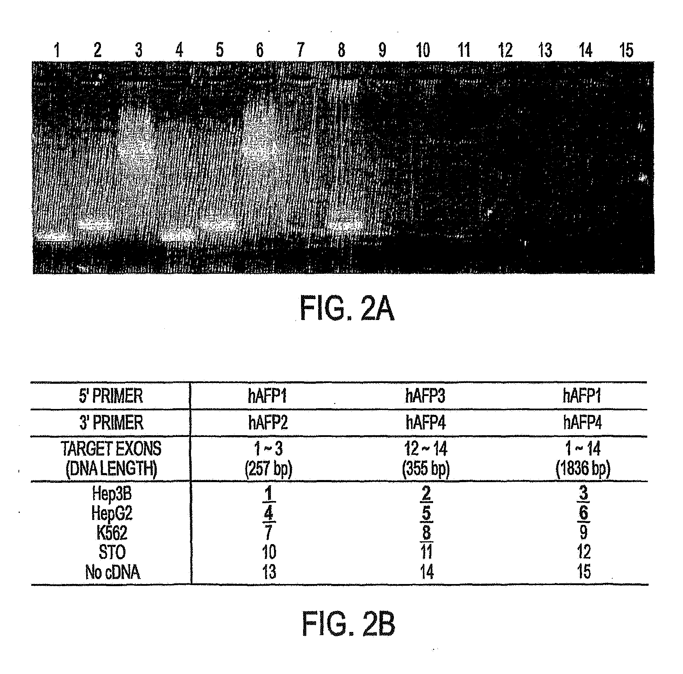

Figures 2a and 2b illustrate PCR analysis of truncated AFP in hemopoietic cells. RT-PCR is carried out using primer combination of hAFP1, hAFP2, hAFP3, and hAFP4. Lanes 1-3 correspond to Hep3B cells; lanes 10-12 correspond to STO cells; lanes 13-15 have no RNA or cDNA. Note, there is a shared band, a truncated AFP isoform, in lanes 2, 5, and 8. There is a truncated AFP isoform unique to liver cells noted in lanes 1 and 4. The complete AFP species is observed in lanes 3 and 6.

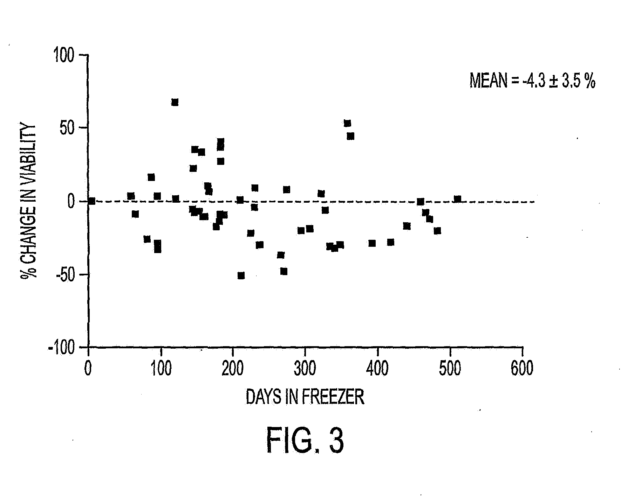

Figure 3 illustrates the relationship between storage time at -170°C and viability of thawed fetal liver cells. Data are expressed as the percent change in viability measured at the time of processing versus the time of thawing. These data indicate that the cryopreservation methods did not significantly affect the viability of the cells. There was no significant change in viability over a period extending to 550 days in storage.

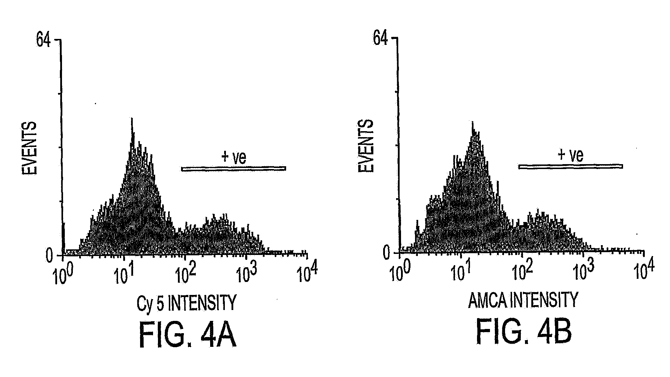

Figures 4a and 4b illustrates typical univariate histograms of fetal liver cell suspensions analyzed by fluorescence activated cell sorting (FACS). The cell suspension was prepared for immunofluorescence analysis of alpha-fetoprotein (AFP) using antibodies conjugated to the red dye, Cy5, and for albumin using antibodies conjugated to the blue dye (AMCA). Thirty thousand cells were screened for red (AFP) and blue (albumin) fluorescence. The results show a clear group of cells positive for each protein. Further analysis shows that about 80 % of the positive populations for each protein are represented by the same cells (i.e. co-expression of the two proteins).

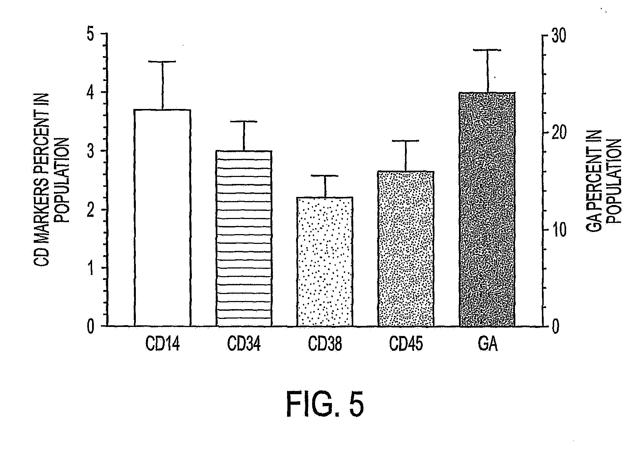

Figure 5 illustrates the percent of cells expressing surface markers CD 14, CD34, CD38, CD45 and Glycophorin A (GA) in unfractionated liver cell suspensions. Note that the GA data is plotted on the right axis to preserve scale.

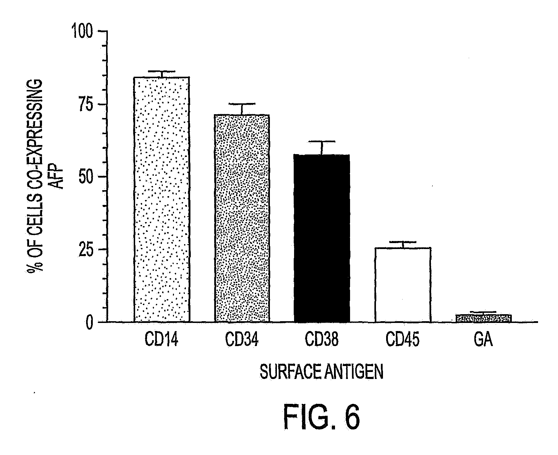

Figure 6 illustrates the percentage of cells in the original cell suspension expressing alpha-fetoprotein and other antigenic markers. Mean ± SEM for percent of cells positive for alpha-fetoprotein (AFP) and specific cell surface markers (CD 14, 34, 38, 45 and glycophorin A).

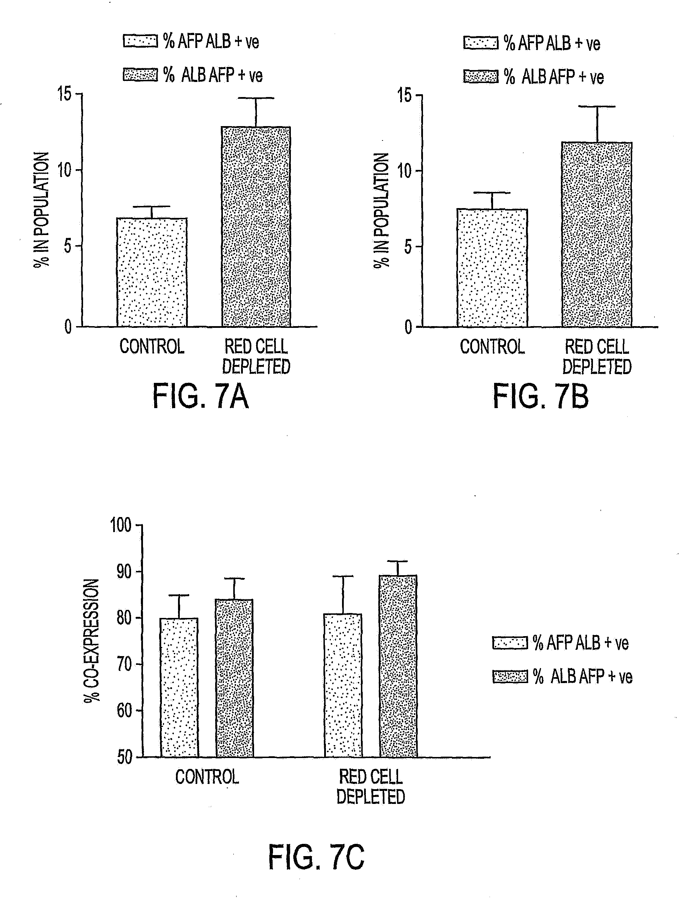

Figures 7a, 7b and 7c illustrate alpha-fetoprotein expression before and after depletion of red blood cells. Figure 7a illustrates the expression of alpha-fetoprotein and Figure 7b illustrates albumin, in suspensions of fetal liver cells with or without selective depletion of red cells using Percoll fractionation. Figure 7c illustrates the proportion of cells expressing both alpha-fetoprotein and albumin, expressed as a percentage of AFP or albumin positive cells. Data for cells with red cell depletion are shown using Percoll fractionation.

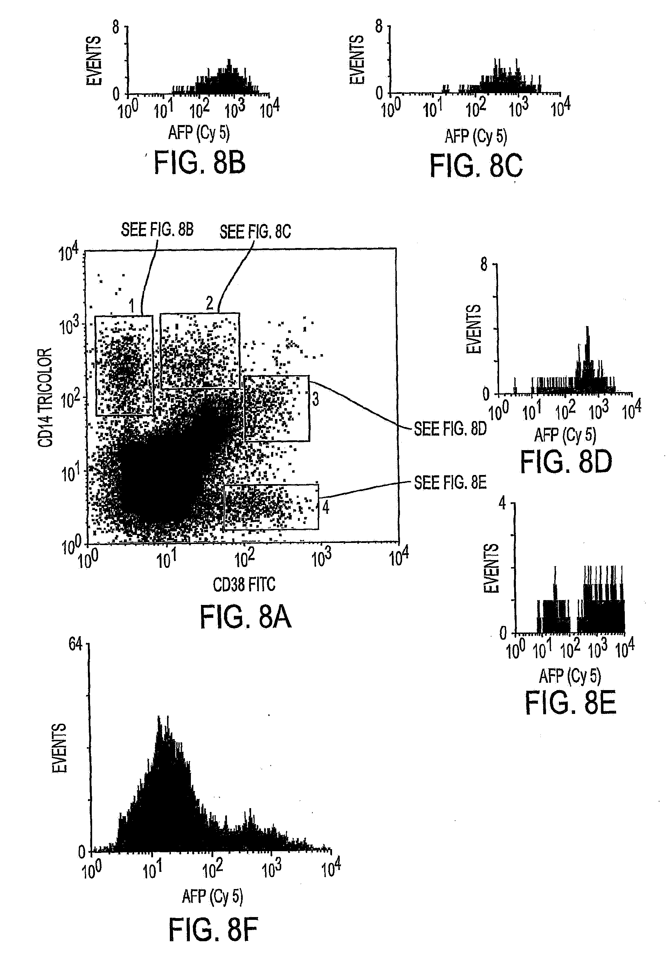

Figures 8a, 8b, 8c, 8d, 8e and 8f illustrate FACS analysis of fetal liver cell suspension for co-expression of CD14, CD38 and AFP. The bivariate scattergram (8a) shows the distribution of TriColor staining for CD14 (ordinate) versus FITC staining for CD38 (abscissa). Gates were created to select specific cell groupings according to the CD14 and CD38 signals. These were then used to display the intensity of AFP staining in each of these subgroups (figures 8b, 8c, 8d and 8e). The AFP results show that a high level of enrichment for AFP is produced by selecting cells positive for either CD38 or CD14. The AFP signal generated from the entire cell suspension (30,000 cells) is shown in Figure 8f.

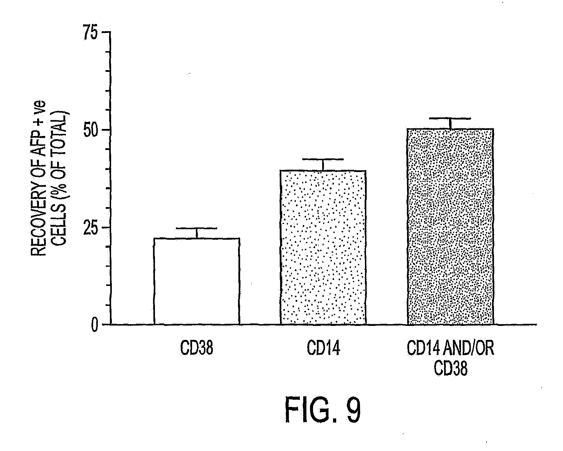

Figure 9 illustrates CD14 and CD38 enrichment for AFP-positive cells. The proportion of AFP-positive cells in cell suspensions prepared from fetal liver can be enhanced dramatically by selecting cells with positive surface labeling for the markers CD38 and CD14. The combination of the two markers produces a significantly better enrichment of AFP-containing cells than that obtained with either marker alone.

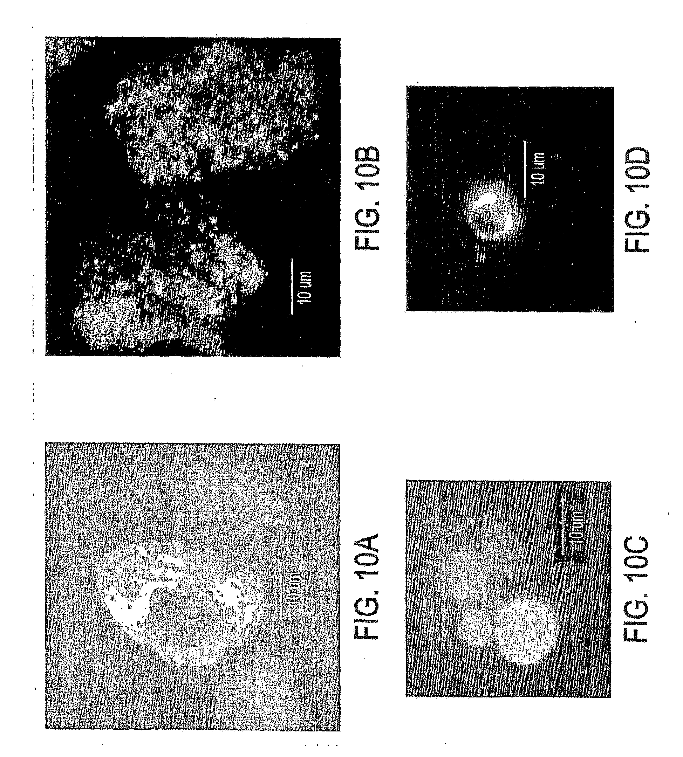

Figures 10a, 10b, 10c and 10d illustrate fluorescence microscopy of human hepatic progenitor cells. Representative hepatic progenitor cells from the fetal liver stained for AFP content. Cell sizes indicate that both early progenitors and more advanced hepatic progenitors are present.

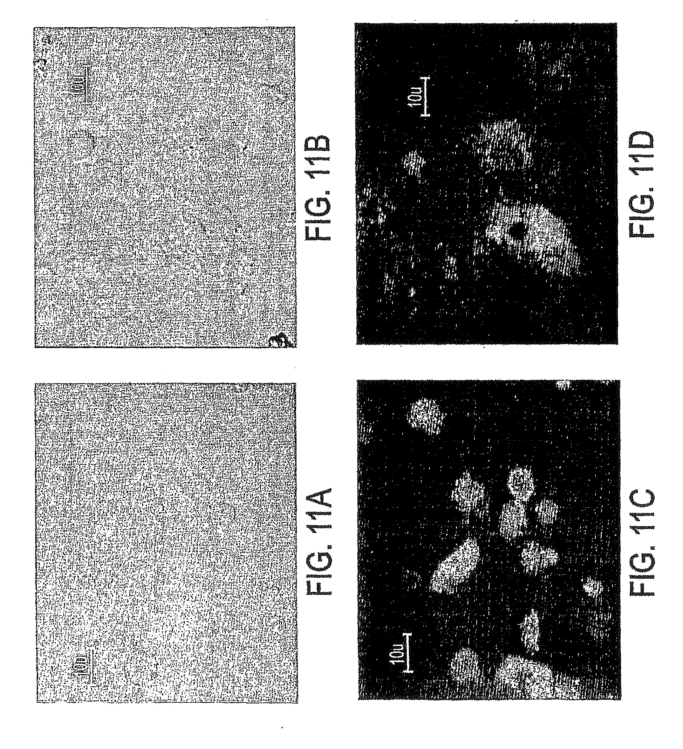

Figures 11a, 11b, 11c and 11d illustrate representative cells selected by expression of AFP. The cells with positive staining for CD14 (11b and 11d) are characteristic of hepatoblasts. The cells with negative staining for surface markers (Figures 11a and 11c) are smaller and consistent in size and morphology with early hepatic progenitors.

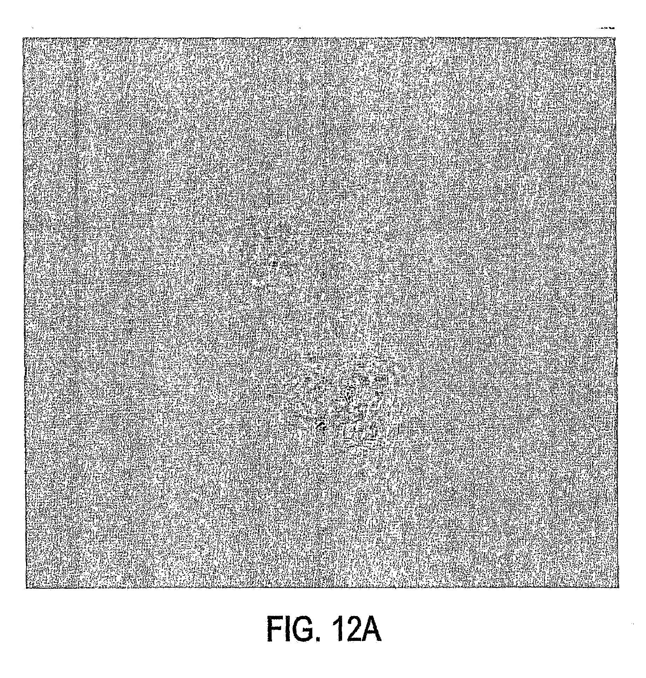

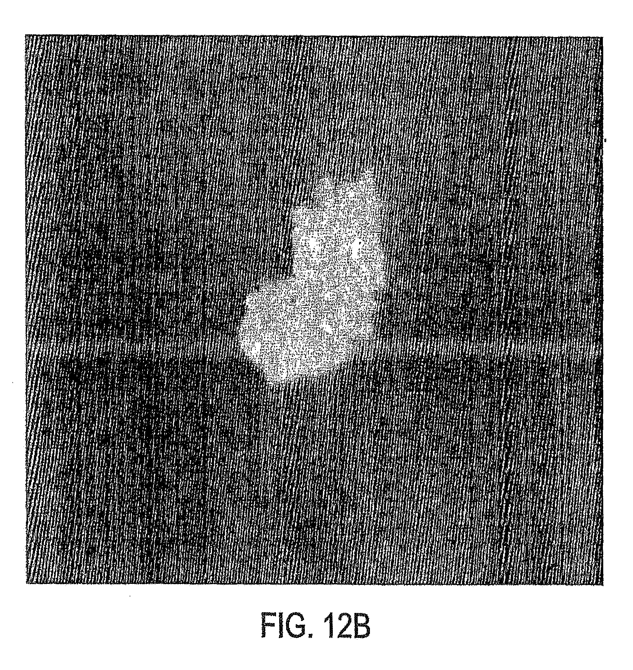

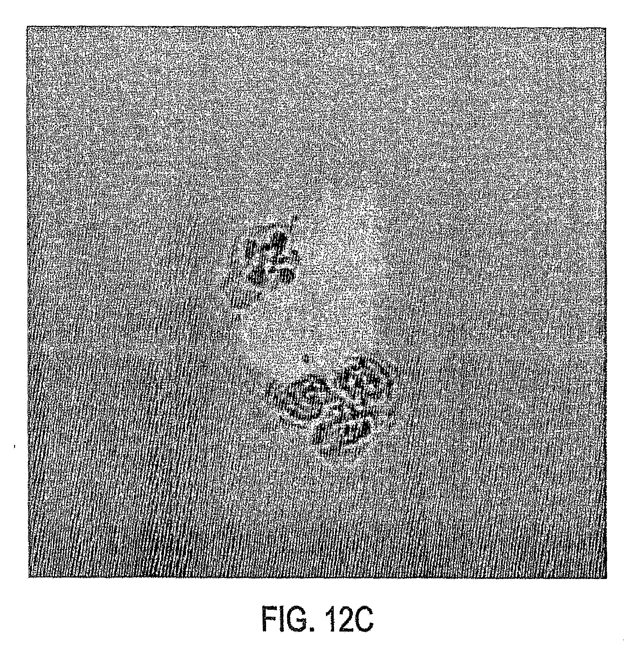

Figures 12a through 12c are of the same field and show that there are two AFP positive cells in this field. Figure 12a illustrates a confocal phase image. Figure 12b illustrates immunofluorescence with antibody to AFP. Figure 12c illustrates overlay (a) and (b) indicating the morphology of AFP positive cells in a group of liver cells

AFP-positive cells are found to have a similar cell size and morphology whether isolated from fetal or adult livers.

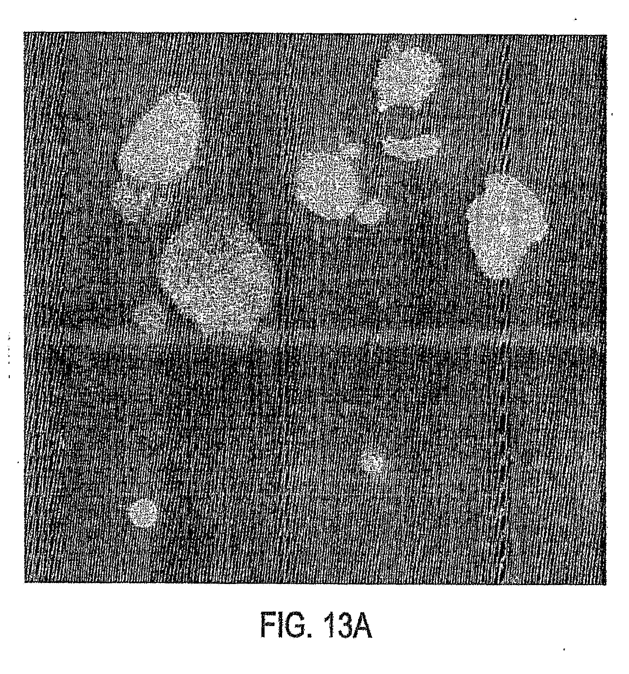

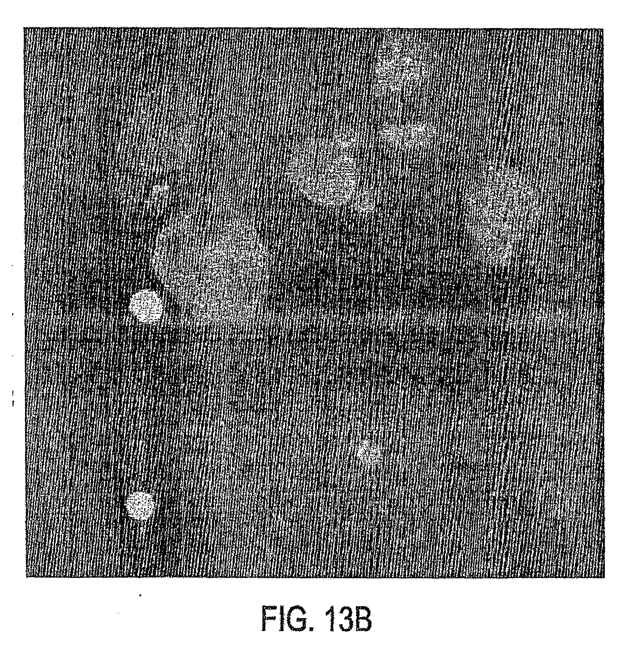

Figures 13a and 13b illustrates cells that are labeled with calcein (a) to show all cell types. Figure 13 (b) consist of the same cells co-expressing AFP and showing that only two cells are AFP- positive. Cell size is not a factor for AFP positivity.

The ability of the liver to regenerate is widely acknowledged, and this usually is accomplished by the entry of normally proliferatively quiescent hepatocytes into the cell cycle. However, when hepatocyte regeneration is impaired, small bile ducts proliferate and invade into the adjacent hepatocyte parenchyma. In humans and experimental animals these ductal cells are referred to as oval cells, and their association with defective regeneration has led to the belief that they are transformed stem or progenitor cells. These cells are of great biological interest since their normal counterparts, the hepatic progenitors can be used as alternative to liver transplants and they can also be useful vehicles for gene therapy for the correction of inborn errors of metabolism. While the ability of progenitors to differentiate into hepatocytes has been demonstrated unequivocally the demand for said cells has not met the desired supply due to the paucity of donor liver tissue.

Isolation of liver progenitors from cadaver human liver, as disclosed herein, is novel and unexpected due to the prevailing opinion in the art that liver loses its utility due to ischemia.

The isolation of human hepatic progenitors from cadaver donors as described herein was obtained through application of a combination of unique methods, markers and parameters which the present inventors used for the first time from cadavers to achieve the unique cell population of this invention.

Alpha-fetoprotein and albumin, both cytoplasmic proteins, are considered to be especially reliable markers for hepatic lineages. They have been the foundation of the strategy to identify the hepatic subpopulations from other cell types in the liver. Both are critical guides in the identification of hepatic cells, but alpha-fetoprotein is especially diagnostic of the hepatic progenitor cells after their purification by flow cytometry. Alpha-fetoprotein, AFP, has been adopted also to estimate the purity of hepatic progenitors after any kind of fractionation strategy.

However, in rigorous controls to prove the validity of these two markers in identifying hepatic lineages, PCR analyses were done to detect expression in multiple cell types that are known to be in liver tissue. PCR analyses are the most sensitive assays detecting even tiny amounts of expression of particular mRNA species. The invention as disclosed herein demonstrates that specific isoforms of both AFP and albumin mRNA can be found in hemopoietic progenitors meaning that when such sensitive assays are used, additional criteria, such as the use of an exon 1 probe for AFP, must be used to define hepatic from hemopoietic cell populations. Although the PCR analyses revealed that hemopoietic progenitors can express both AFP and albumin mRNA species, the mRNA expression levels are very small. Indeed, when AFP and albumin are measured at protein levels, no detectable AFP or albumin could be found in the hemopoietic progenitors. Therefore, for routine protein assays (immunofluorescence, Western blots, etc.) and for assays of high level expression of mRNA (Northern blots), AFP and albumin remain as valuable markers defining hepatic lineages.

This invention also discloses the design and preparation of specific primers of RT-PCR to determine the expression pattern of AFP mRNA isoforms in hepatic versus hemopoietic cell populations. Three different combinations of primers for human AFP RT-PCR were used in distinguishing AFP mRNA expression in hepatic and hemopoietic lineages. To test the expression of AFP in hemopoietic cells, as exemplified in Example 1, the inventors have screened several lines of hepatic versus hemopoietic origin for complete versus truncated forms of the AFP. RT-PCR, which is the most sensitive technique known for identifying particular RNA templates, is used in these studies. The data thus far indicates that human AFP is present in a complete form in two human cell lines (HepG2 and Hep3B) derived from hepatic progenitors and in a truncated form in a human cell line, K562, derived from a hemopoietic progenitor cells. The fact that exon 1 is unique to hepatic progenitor subpopulations enables one to use it as a probe for identifying hepatic versus hemopoietic progenitor cell types. This test is used to identify specific subpopulations of liver progenitor cells of this invention.

Accordingly, the inventors have designed nine PCR primers in order to detect the presence of human AFP mRNA in liver progenitors. All the primer combinations detect AFP mRNA in human hepatic cell lines HepG2 and Hep3B. However, all primer combinations other than one for full-length hAFP mRNA amplify the portion of the AFP mRNA in a human erythroleukemia cell line, K562. As predicted above, this demonstrates that one of the truncated forms of AFP, but not the full-length one, is expressed in K562. The result suggests that the only useful primers for identifying hepatic cells are those that detect the full-length AFP, the expression of which is more provably restricted in a tissue-specific manner. Several lines and primary tissue of hepatic versus hemopoietic origin are screened for complete versus truncated forms of the AFP. Although a truncated form of AFP is found in some hemopoietic tissues, it is unknown which cell type within the tissue was expressing it.

Because a truncated form of AFP is found in some subpopulations of hemopoietic cells, albumin is also analyzed in both hepatic and hemopoietic cells. Primers for albumin are developed in a fashion analogous to that for AFP (see above) and used to assess albumin expression in hepatic versus hemopoietic cell lines (see Figure 4). As for AFP, a truncated form is found in K562, the hemopoietic cell line, and a transcript that could be detected by the primer for exon 12-14.

Prior to the studies described herein, and in the vast literature on hemopoietic progenitors, no one has ever reported expression of mature or truncated AFP or albumin in normal hemopoietic progenitors in human.

### Processing and Cryopreservation of Human Liver Progenitors

In order to optimally yield dissociated human liver progenitors from fetal or adult livers, the protocol disclosed herein makes use of the upper fractions of a density gradient and excludes the pellet. The novel variation to the density gradient centrifugation, as disclosed herein, is that the pellet is discarded and cells with a lower buoyant density (i.e., cells collecting at the top of the gradient) are retained and used for further studies. The inventors have found that younger cells (i.e. diploid) and cells more robust to cryopreservation are present at the top of or within the Percoll density gradient.

The culture methodologies as described herein are unique and are modified further for human and/or rodent liver cells. Additionally, the cultures can include biodegradable beads coated with purified extracellular matrix components, and could then be used to inoculate the cells bound onto the beads for use in bioreactors.

Cryopreservation methodologies of this invention are unique and distinct from the methods used in the prior art. Major distinctions are due to the use of different buffers and cryopreservation of a diploid hepatic cell population that can include a progenitor population which is low in density and thus, buoyant in gradient centrifugation.

Successful cryopreservation of mature human liver cells is highly desired but has never been achieved in the art. Generally, successful cryopreservation is defined as the ability to freeze the cells at liquid nitrogen temperatures (-160 to 180°C) and then to thaw them and observe viabilities of >75% and with the ability to attach onto dishes. Cell lines of any origin, such as sperm and ova and cells from fetal tissues, can be frozen successfully in an aqueous buffer (i.e. a medium such as DME, Dulbecco's Modified Eagle's medium, or RPMI 1640) and supplemented with 10% serum + a cryopreservative (most commonly dimethylsulfoxide: DMSO) and yield viabilities at thawing of 70-90% and with excellent ability to attach.

The special cryopreservation methodology of this invention is achieved through the use of a novel buffer, a novel cell population, and a variation of this that includes embedding the cells in forms of extracellular matrix. This methodology for the first time achieves viability upon thawing that is not different from the viability measured prior to freezing, immediately after cell dispersion (See Figure 3). Actual viabilities are variable due to the condition of the tissue upon arrival and, in the present studies, averaged 77% for the cadaveric fetal liver cells. The cryopreservation methodologies results in no significant loss in viability by the freezing process and in cells that could attach and expand ex vivo after thawing.

### Cell Markers and Flow Cytometry

Using our current definition of liver progenitors as immature cell populations that express alpha-fetoprotein with or without expression of albumin, markers are assessed that will specifically select these cells. A startling discovery is that many of the markers (i.e. CD34) that are classical ones for hemopoietic progenitors, also identify hepatic progenitor subpopulations. Thus, single color sorts for CD34 resulted in significant enrichment (at least 9-fold) for cells that express AFP. However, not all of these AFP-positive cells can be verified to be hepatic progenitors. Based on the percentage that are albumin positive, about 80-90% of the cells are hepatic progenitors, and the others are either hepatic progenitors too immature to yet express albumin or possibly hemopoietic subpopulations that express alpha-fetoprotein.

This invention uses a unique flow cytometric sorting strategy. Using the combination of AFP and albumin expression, as two uniquely defining features of hepatic progenitors, antigenic markers and other flow cytometric parameters are identified that define the hepatic progenitor cells. The sorting strategies to date involve sorts for small cells (< 15 micron by measures of forward scatter), that are diploid (using fluorescence from Hoechst dye 33342), are agranular by side scatter, are negative for certain hemopoietic antigens (i.e. glycophorin A, the red blood cell antigen and CD45) followed by or proceeded by positive markers shared between hepatic cell subpopulations and hemopoietic cell subpopulations (i.e. CD14 and/or CD38.)

In the experiments described herein, the inventors identify hepatic progenitor cells by sorting for those cells that strongly express alpha-fetoprotein, express CD 34, which is known to be a specific hemopoietic stem cell marker, and optionally weakly express albumin. Also described herein, is the evidence that hemopoietic cells can also express AFP, albeit a truncated form. The inventors have described a novel cell population and process of isolation, identification, culture, and a method of using such cell population. The success in the isolation, identification, and culture of the particular cell population of the invention is achieved partly through advanced methods of isolation, affinity debulking, high-speed fluorescence-activated cell sorting, having greater speed and accuracy, and modified cryopreservation and culture techniques.

Flow cytometric sorting strategies are devised to purify liver progenitors from freshly isolated cell suspensions or from thawed cryopreserved liver cells and that involve 1) staining of the cells with several fluroprobe-labeled antibodies to specific cell surface markers and 2) using a combination of negative and positive sorting strategies in multiparametric flow cytometric technologies. The methods for purification of specific lineage stages from human hepatic cell populations can be used with livers from any age donor, since the markers appear to be lineage-position specific.

The improved methods of labeling the cells, and a dramatically improved flow cytometer ("a MoFlo" flow cytometer from Cytomation which sorts cells at 40,000 cells/second and performs 8 color sorts) over that which was used in the past (Becton Dickenson's FACSTAR PLUS which sorts cells at 2000-6000 cells/second and performs 2-4 color sorts;) assist in the successful isolation, and identification of this novel cell population.

The expression of AFP and albumin like immunoreactivity is well defined in the cell suspensions, with a clear group of cells showing a clear differentiation from the background signal (Figure 6). Alpha-fetoprotein is expressed in 6.9 ± 0.86% of cells in unfractionated cell suspensions while albumin was present in 7.7 ± 1.1%, Among AFP positive cells 75.6 ± 4.9% co-expressed albumin while 80 ± 5.5 % of albumen positive cells also expressed AFP. Thus, approximately 25% of cells expressing alpha-fetoprotein did not express albumin and 20% of cells expressing albumin did not express alpha-fetoprotein.

The proportion of cells bearing the principle surface markers used in this work are shown for complete cell suspensions (i.e. including red cells) in Table 1 (where GA is glycophorin A, a surface marker on red blood cells):

**Table 1: Percentage of CD Positive Populations In Original Liver Cell Suspension and Percentage of these that are Positive for AFP**

| | CD14 | CD34 | CD38 | CD45 | GA |
|---|---|---|---|---|---|
| Unfractionated | | | | | |
| % in population | 3.7 ±0.8 (8) | 2.8 ±0.5 | 2.2 ±0.4 | 2.6±0.5 | 36.8±5 |
| % AFP positive | 81.7± 2.2 | 72.6± 4.2 | 57.6 ± 4.6 | 22.2 ± 4.4 | 2.3 ± 0.6 |

Clearly, glycophorin A (GA) positive cells (i.e. erythroid cells) represent a major component of the cell mass but an insignificant fraction of the AFP-positive cells. Thus, when cell suspensions are depleted of red cells by Percoll fractionation the proportion of cells expressing AFP is increased significantly to 12.9 ± 1.9% and those expressing albumin to 12.1 ± 2.3%. The percent of AFP positive cells co-expressing albumin is also increased to 80.5+8.2% and the proportion of albumin-positive cells co-expressing AFP increased to 89+3.1%, though neither change is statistically significant. The result of this procedure on the proportion of cells bearing the surface markers are shown in Table 2, together with the proportion of each subgroup showing positive staining for AFP.

**Table 2: Percentage of CD Positive Populations in Liver Cell Suspension after Depletion of Red Cells and Percentage of these that are Positive for AFP**

| | CD14. | CD34 | CD38 | CD45 | GA |
|---|---|---|---|---|---|
| Red cell depleted | | | | | |
| % in population | 7.4 ± 1.3 | 3.4 ± 0.5 | 4.8 ± 0.9 | 8.2 ± 0.3 | 27.5 ± 4.7 |
| % AFP positive | 89.8 ± 1.3 | 77.1 ± 2.9 | 53.5 ± 7.2 | 32.5 ± 1.3 | 1.8 ± 0.9 |

In most cases, the presence of AFP in the subgroups selected by cell surface marker is distributed continuously with a clear preponderance of cells showing staining intensities in the positive range. However, the distribution of CD38 positive cells with respect to co-expression of AFP is unique. In CD38-positive cells a bimodal distribution for AFP co-expression is apparent in which two distinct groups of cells are apparent, one group positive for AFP, the other negative. This is illustrated in Figure 8a which shows a scattergram of cells stained for expression of CD14 and CD38 together with univariate histograms of alpha-fetoprotein expression in cells positive for CD 14 and/or CD 38.

The results show that alpha-fetoprotein (AFP) is present in 7% of the cells in single cell suspensions of fetal liver tissue (i.e. in the original cell suspension). The antibody to glycophorin A (an antigen on red blood cells, erythrocytes) is found to identify a subpopulation of cells that did not express AFP. Thus, cells expressing this antigen (i.e. erythroid cells) are excluded from cells intended for characterization of hepatic progenitors. The CD38 antigen identified a population of cells that shows significant enhancement in the proportion of AFP positive cells (i.e., greater than 7 times the proportion in unfractionated samples. Both antigens have a number of isoforms, depending on whether or not there are sections of the molecule, encoded by splicing variants, present. Antibodies are available that identify the various isoforms.

The classic marker for hemopoietic progenitor cells, CD34, is present on many cells that also express AFP. The sorting of cells positive for CD34 results in enrichment of AFP-positive cells at least 9 fold over that found in the original cell suspension (67%, in the CD34-positive cells vs 7% in the original cell suspension). However, the most effective single antibody for enrichment of AFP positive cells is CD 14, which produces a greater than 11 fold increase in the proportion of these cells compared to the original population (81% versus 7%).

Accordingly, the yield of AFP-positive cells is improved by using a combination of surface markers. Thus, the extent of co-expression of AFP with selected combinations of surface markers is determined to establish the extent to which the selection the intracellular marker is increased. The data are expressed as the proportion of AFP-positive cells expressing surface markers (termed the "yield" of AFP-positive cells) and as the proportion of all AFP-positive cells that appear in the population defined by the surface marker (termed the "enrichment" factor for AFP positive cells). Results for combinations of CD14, CD34 and CD38 are shown in Table 3 together with the results from individual markers for comparison.

**Table 3.**

| | CD14 | CD34 | CD38 | CD14 ± CD38 | CD14 ± CD34 |
|---|---|---|---|---|---|
| Enrichment | 80.6 ± 2.6 | 66.7 ± 4.7 | 53.8 ± 4.5 | 66.9 ± 3.5 | 68.2 ± 3.9 |
| Yield | 39.8 ± 2.6 | 26.9 ± 4.4 | 22.0 ± 2.7 | 50.6 ± 2.7 | 52.2 ± 5.5 |

Enrichment. Percent of cells expressing either (or both) of the surface markers that are also positive for AFP.

### Yield. Percent of all AFP-positive cells that also expressed one or both of the surface marker combination

These data are also shown for the CD14/CD38 combination of markers in Figure 9

The morphology of cells staining positive for AFP is variable and encompassed the entire range of cell size and shape in the cell suspension from fetal livers but not adult liver. The largest of the AFP-positive cells, approximately 12-15 micron, is much smaller than mature hepatocytes, ranging in size from 20-50 micron). This is illustrated in Figure 10, which shows several AFP-positive cells selected for the expression of specific antibodies.

In all cases a certain proportion of AFP-positive cells show no expression of any surface antibodies used in this study. The appearance of these AFP-positive "null" cells is illustrated in Figures 11a and 11c where they are compared with the appearance of CD14 positive/AFP positive cells (Figures 11b and 11d) sorted from the same suspension. The Figures 11a and 11b are differential interference contrast microscopy and the Figures 11c and 11d are AFP immunofluorescence. It is clear that while both cell types are positive for AFP, the cells staining negative for surface antigens are consistently smaller and less complex than the CD14 positive cells.

In summary, the markers for sorting hepatic progenitors are Glycophorin A⁻, CD45⁻, ICAM⁺, CD14⁺ and/or CD38⁺, or null for all these markers but ICAM⁺, diploid, agranular (by side scatter), less than 15 microns (by forward scatter). The phenotype of these sorted cells is small cells (< 15 microns), with little cytoplasm (high nucleus/cytoplasmic ratio), albumin and/or AFP⁺⁺⁺. For morphology of the cells see Figures 10-12.

### Confocal Characterization of alpha-fetoprotein-Expressing Cells in Fetal and Adult Human Liver.

Confocal microscopy is used to obtain the images from human fetal, pediatric, or adult cells that express alpha-fetoprotein. This methodology enables one to observe the morphology and size of these cells and to show directly the location of intracellular proteins, such as AFP and ALB, and that of membrane surface markers such as CD34 and CD38. AFP-expressing cells are found in both fetal, pediatric, and adult livers (Figure 12a). Fetal livers, as expected, have the highest percentage (6-7%), whereas adult livers have a small percentage (<4% in young adults) and with the numbers declining with age to <1% in middle-age adults. No AFP-expressing cells have been found in a liver from donors older than 57 years of age. The few hepatic progenitors found in cadaveric livers are enriched significantly through the Percoll fractionation process to yield up to 2% of the cells in Percoll fractions 1 and 2 from the donor livers (Table 4).

Table 4 shows the cell size and viability from fractions of Percoll-supplemented buffers. Smaller cells (fractions 1-3) have higher viability than larger cells (fraction 4) after being isolated under the same condition.

**Table 4.**

| Percoll | | | |
|---|---|---|---|
| Fraction | Viability(%) | Cell Size (µm) % | AFP⁺ cells |
| Fraction 1 | 82 | < 12 | 0.5-1 % |
| Fraction 2 | 84 | 10-15 | 2 % |
| Fraction 3 | 85 | 15-25 | < 0.2 % |
| Fraction 4 | 56 | 25-50 | < 0.01% |

These results indicate that donor organs preferably useful for liver cell therapies as well as for organ transplantation include those from young donors up to 45 years of age and such livers are preferably isolated within the first 30 hours from heart arrest. The livers from geriatric patients (>71 years of age) are inappropriate donors for cell therapies and perhaps also for whole organ transplants, especially for children, since they will have little if any regenerative capacity from hepatic progenitors and only the minimal regenerative capacity known to be available from the mature cells.

### Maturational Lineage

The inventors of this invention have shown that ischemically damaged livers contain a hepatic progenitor cell population capable of growth and differentiation into hepatocytes and biliary cells under both normal and disease conditions. This invention stands for the proposition that every position in the liver lineage is a distinct maturational stage, and that there are multiple stem cell populations in the liver.

Surprisingly the liver of the instant invention provides 3 separate maturational lineages: one responsible for hepatopoiesis, yielding liver tissue and with cell fates of hepatocytes and biliary cells (bile duct); another for hemopoiesis, yielding blood cells with cell fates of lymphocytes (B and T), platelets, macrophages, neutrophils, and granulocytes; and a third for mesengenesis, yielding mesenchymal derivatives and with cell fates of endothelia, fat cells, stromal cells, cartilage, and even bone.

The isolated cell population of this invention has great potential as successful liver-directed cell and/or gene therapy. This invention, as described in the Examples, has made substantial advances in identifying conditions in which nonhuman primate as well as human hepatic progenitors can be successfully placed into cell culture and maintained while still retaining their capability to fully differentiate or to mature. Following the teachings disclosed herein, it is possible to isolate from cadavers and maintain undifferentiated hepatic progenitors in culture and then switch them to a differentiation-associated media for transplantation.

Because of the ability to significantly expand in vitro, the cell population of this invention, similar to cells in hemopoietic lineage, can be used as a cell seed for ex vivo expansion. This would eliminate the necessity for major invasive surgical resection of the patient's liver.

Once the human hepatic progenitors are established in culture, gene transfer is performed. This is accomplished with a number of different gene delivery vector systems (see Example provided infra). An important consideration at this point is that some forms of gene transfer require rapidly growing cells, and since human diploid cells and/or progenitors of the invention significantly divide under normal physiological conditions, these cells are ideal candidates for gene transfer to liver. Also, the growing characteristics of the cell population of this invention permits the use in an ex vivo gene transfer using certain gene delivery viral vectors that will require cell proliferation for efficient gene insertion and expression.

The progenitor cell population of this invention is also suitable for an autologous or allogeneic liver-directed cell or gene therapy. Clearly, the use of autologous hepatic progenitors will eliminate a. significant concern regarding rejection of the transplanted cells. The cell population of this invention is particularly attractive for allogenic cell transfer, because their antigenic profile suggests minimal immunological rejection phenomena. Moreover, other cellular elements, such as blood cells, endothelial cells, Kupffer cells, that are known to be highly immunogenic are substantially eliminated through the purification process.

Once the autologous or allogenic hepatic progenitors are isolated and purified, they are be genetically modified or used intact, expanded *in vitro* if need be and then transplanted back into the host. If genetic modification is desired, after genetic modification and before transplantation, those genetically modified cells can be expanded and/or selected based on the incorporation and expression of a dominate selectable marker. Transplantation can be back into the hepatic compartment or a heterotopic site. For transplantation into the hepatic compartment, portal vein infusion or intrasplenic injection could be used. Intrasplenic injection can be the administration route of choice because most of the hepatic progenitors transplanted via an intrasplenic injection move into the liver. Once in the hepatic compartment, the transplanted, genetically modified hepatic progenitors mature to a normal hepatocyte morphology.

Additional medical procedures can assist in the efficacy of hepatic engraftment of the transplanted hepatic progenitors. Animal models have demonstrated that in partial hepatectomy administration of angiogenesis factors and other growth factors aide in the engraftment and viability of the transplanted hepatocytes. An alternative approach is to transplant the genetically modified hepatocytes to a heterotopic site.

To date, the cell therapy approaches with respect to liver have shown only modest efficacy. This can be due to the fact that the donor cells being used are predominantly adult liver cells and are short-lived after isolation and reinjection. In addition, the use of adult cells results in strong immunological rejection. In the instant case the hepatic progenitor cells offer greater efficacy because of their limited capacity to elicit immunological rejection phenomena and because of their extensive regenerative potential.

With respect to gene therapy, the ongoing efforts make use of "targeted injectable vectors", the most popular route for clinical therapies under development. These approaches have had limited efficacy due both to immunological problems and transient expression of the vectors. Ex vivo gene therapy with progenitor cells (or use of injectable vectors somehow targeted to those progenitor cell populations) may prove more effective, since the vectors can be introduced ex vivo into purified subpopulations of the progenitor cells; the modified cells selected and reintroduced in vivo. The advantages of the progenitor cells are their enormous expansion potential, their minimal, if any, induction of immunological reactions, and their ability to differentiate to produce the entire lineage of mature cells.

### Common or Interdependent Lineages

The improved methodologies enable the inventors to more closely study and characterize hepatic progenitors. These studies reveal a specially close relationship between hepatic progenitors and hemopoietic progenitors indicating a close relationship between these two lineages. Indeed, these studies show that the progenitor cells of the hepatic and hemopoietic lineages share numerous antigenic markers (CD14, CD34, CD38, c-kit, oval cell antigens), share biochemical properties (i.e. transferrins, glutathione-S-transferases, and a truncated isoform of alpha-fetoprotein), and have extensive overlap in the culture requirements (forms of extracellular matrix and specific hormonal requirements) for expansion ex vivo. The progenitor cells of both lineages are located in the same sites within the liver acinus. Finally, paracrine signaling is present throughout the cells of the two maturational lineages; that is signals produced by each of the lineages regulates cells in the other lineage. Indeed, it is concluded that there is a common lineage or at the very least interdependent lineages between the hepatic and hemopoietic cells.

The cell populations disclosed herein can be purified and utilized to yield either myelo-hemopoietic cells or hepatic derivatives depending on the conditions under which the cells are isolated and cultured. Thus, if the cells are reintroduced in vivo into blood, they could potentially give rise to myelo-hemopoietic derivatives; if introduced into liver, they should yield liver cells. Parallel phenomena should occur in cells maintained ex vivo. Therefore, bioreactor systems inoculated with cell populations sorted for a set of antigens that defines both hepatic and hemopoietic progenitors (i.e. CD38⁺, c-kit⁺, CD45⁻) can result in cell populations with multiple fates.

Another important aspect of the cell population of this invention is that some of the cells in the population display a specific progenitor cell surface antigen CD34. CD34 has been used as a convenient positive selection marker for hemopoietic stem cells of bone marrow. This invention, as disclosed herein, suggests better ways to purify any progenitor population, such as the hemopoietic and the hepatic progenitor cell populations which can subsequently be used in the clinical and pre-clinical programs.

The uses for human hepatic progenitors are many and diverse. They include: 1) research on human cells; 2) production of vaccines or antivirals; 3) toxicological studies; 4) drug development; 5) protein manufacturing (using the cells as hosts for various human-specific factors); 6) liver cell therapies; 7) liver gene therapies; 8) bioartificial livers that can be used in research, toxicological and antimicrobial studies, protein manufacturing, or clinically as a liver assist system. Considering the possibility of a common lineage between hemopoiesis and hepatopoiesis, as advanced by the inventors of this invention, the same cells are suitable both for hepatic or hemopoietic fates depending upon the microenvironment in which they are placed.

The availability of highly purified human hepatic progenitor cells enable much more extensive research on human cells, and will certainly facilitate the development of successful forms of liver cell and gene therapy, and enable the development of human bioartificial livers for use both in research and as clinical assist devices. At present, the limited supply of healthy human tissues precludes clinical programs in liver cell therapy or in human bioartificial livers. The diploid cells, including their progenitor cell subpopulations obtained from cadavers, have sufficient expansion potential to greatly alleviate that limited supply.

The following examples are illustrative and are not intended to be limiting.

### 6. Examples

### 6.1 Procurement of Livers from Cadavers

The livers from cadavers are catheterized by the portal vein, vena cava, or by both, perfused with buffers to eliminate blood; and then perfused with buffers containing collagenases/proteases to enzymatically dissociate the cells. After the digestion, taking usually 15-30 minutes depending on the size of the liver, the tissue is pressed through cheesecloth or a nylon filter or raked with a comb to mechanically complete the cell dissociation process. The dissociated cells are rinsed with a buffer containing serum to inactivate the collagenase and other enzymes used in the perfusion process.

The perfusion buffers, P1 and P2, are placed in a water bath at 37°C. The perfusion is carried out in a Miller type perfusion box, which is maintained at 37°C throughout the perfusion. The buffers are oxygenated during the perfusion. All tubing in the box is rinsed with 70% ethanol, followed by distilled water and then with PI to ensure that the air has been removed from the system. The liver is cannulated using a Teflon cannula from a 16-gauge needle attached to 60 ml syringe to flush ice-cold PI buffer through the liver using various blood vessels available on the cut surface of the liver for large pieces of liver (100-300 g). For the cases when an entire liver lobe becomes available, the remnants of the vena cava can be cannulated. The various blood vessels in chunks of liver are tested to learn which will offer optimal perfusion of the tissue. This procedure also removes any excess blood from the liver. The chosen blood vessel is cannulated and sealed into place using medical grade adhesive (e.g. medical grade "superglue"). All other large vessels and surface openings are sealed using the medical grade adhesive, and, if required, using Q-tips with the adhesive to help seal the openings. Once the adhesive has dried, the liver specimen is placed on a nylon mesh within an appropriate size glass bowl. The P1 buffer is added to the bowl, and the liver submerged in the buffer. The bowl containing the liver is placed inside the perfusion box, and the outlet tubing of the cannula is attached. The P1 buffer is recirculated for 15 minutes starting at a low speed of about 24 mls/min and then slowly increased to between 58 ml/min and 90 ml/minute to optimize a flow rate with an acceptable back pressure. One must check that there are no excessive leaks of the perfusate from the liver. After 15 minutes, the P1 buffer is removed from the bowl and replaced with the P2 buffer containing the collagenase. The P2 buffer is recirculated until the liver is sufficiently digested (evaluated by color-conversion of liver from dark reddish brown to pale brown and by acquisition of mushy texture to liver). The P2 buffer is recirculated for no longer than 20-25 minutes. Once the perfusion has ended, the P2 buffer is drained from the bowl and the liver transferred in the bowl to a biological hood.

The cell culture medium (DMEM) is added to the bowl, and the cannula and the adhesive is removed along with any undigested regions of the liver. The capsule of the liver (Glisson's capsule) is broken using tissue forceps and scissors. This allows the release of the digested tissue into the medium leaving behind the connective tissue and any undigested material. The digested material is put into the DMEM and then filtered through a series of different size filters. The filters are placed inside a large funnel to aid the filtration. The digested material is filtered first with a single layer of cheesecloth, followed by a 400 µ nylon filter, and then through a 70 µ Teflon filter. The filtrate is divided equally into centrifuge tubes and centrifuged at 70 g for 4 minutes.

After centrifugation, prior to the addition of Percoll, the supernatant is referred to as the Fraction 1 (F1). To the pellet of cells, DMEM and isotonic Percoll are added to give a final ratio of 3:1 respectively. For example, a small pellet of packed cells of 5 ml volume is suspended in 30 mls of DMEM and 10 mls of isotonic Percoll. The sample is centrifuged at 100 g for 5 minutes to yield a pellet referred to as the F4 fraction. The supernatant is centrifuged again for 5 minutes at 200 to 400 g to obtain a pellet referred to as the F3 fraction. The supernatant is centrifuged again now at 600-800g to obtain a pellet referred to as the F2 fraction. A final centrifugation is done at 1200-1800 g to obtain a pellet referred to as the F1 fraction. The cells of the different fractions are suspended and assessed for viability using the Trypan blue dye exclusion assay. The viabilities of these different fractions are presented in Table 4.

Cells that remain bound to the vascular or biliary tree of the liver tissue following liver perfusion are retained. These cells are found in the original suspension of cells obtained after enzymatic perfusion, and are typically left on the top of the sieves (e.g. cheesecloth) after passing through the cells in suspension. These remnants of the vascular and biliary tree are processed again with enzymes and the resulting cells pooled together with the other cells.

Percoll fractionation is used routinely in liver perfusions by most investigators to eliminate what they assume to be debris and dead cells; only the final pellet is preserved by those investigators. The novel variation to the perfusion routine, as disclosed herein is that the first pellet - here, termed F4 - contains cells found to be the most sensitive to ischemia, whether warm or cold, and cells with a lower buoyant density (i.e., cells collected from pellets obtained at centrifugation at higher speeds) are less sensitive to ischemia. These cells in the F1, F2, and F3 fractions are smaller, presumably younger parenchymal cells and have a much greater ease of freezing (see section on cryopreservation). Moreover, these cells are substantially diploid cells, whereas the cells in the F4 fraction from adults comprises largely polyploid cells. The polyploid cells can be binucleated or can be mononuclear and tetraploid or octaploid, oro even higher levels of ploidy.

### 6.2. Ploidy in Relation to Fractionation of Cell Populations

The fractions of adult liver cells (F1-F4), described above, are found to contain distinct cell populations: F1 contains debris, red blood cells, hepatic stellate cells, and small hepatic cells (<12 µm) that contain progenitor cell populations (of either hepatic or hemopoietic lineages); the F2 fraction contains larger hepatic cells (12-15 µm) that are diploid, small parenchymal cells; the F3 fraction contains yet larger parenchymal cells (15-25 µm) and consisting of a mixture of diploid and tetraploid cells; and the F4 fraction (the one used by all other investigators) consisting of the largest of the parenchymal cells (25-50 µm) and that are almost entirely polyploid (e.g. tetraploid and octaploid).

In general, the parenchymal cells in the F1-F3 fraction have a viability after freezing of 79-95%; the parenchymal cells in the F4 fraction have a 50-80% viability after freezing (depending upon the conditions of the liver upon arrival). The identified variables influencing viability of the parenchymal cells in the F4 fraction are : 1) age of the donor (the older the age of the donor, the worse the prognosis for the cells); 2) the time between heart arrest and delivery to the lab (the shorter the better). These factors are interactive such that rapid delivery of tissue from an older donor can be more attractive than tissue from a young patient that has spent too long in transit.

### 6.3. Effect of Ischemia on Cell Fractionation

Results of cell viability are examined as a function of time and temperature of ischemia. The condition in which the liver is kept at ambient or above temperatures is termed warm ischemia. The condition in which the liver is kept at temperatures below ambient is termed cold ischemia. In common practice, warm ischemia corresponds to a temperature between vital body temperature and room temperature whereas cold ischemia corresponds to any temperature below room temperature, e.g. about 10 °C or about 4 °C.

In warm ischemia, the livers are kept at above ambient temperatures and the livers are then perfused. In an alternate form of warm ischemia, the livers are kept at above ambient temperatures for a time, then chilled, and subsequently perfused with warm dissociation solutions. The single cell suspension resulting from either perfusion is processed to provide cell fractions that have different proportions of diploid and polyploid cells. Progenitors are a subpopulation of diploid cells. Moreover, it is observed that differentiated, polyploid cells are sensitive to ischemia at temperatures above ambient. In the following table, viable rat liver cells are distinguished from dead cells by staining with propidium iodine (PI), which stains nuclei of dead cells. Monocucleated and binucleated cells were counted in live and dead fractions, sorted by flow cytometry after fixation, permeabilization, and restaining with PI to visualize nuclei in all cells.

**Table 5**

| *Duration of warm ischemia* | *Live cells % binucleated* | *Dead cells % binucleated* |
|---|---|---|
| none | 32 | 30 |
| 2 hr | 27 | 47 |

A total cell yield from liver perfusion is measured as a function of time of warm ischemia using rats as a model. Male Sprague-Dawley rats of 250-300 g each, about 8 weeks of age, are used. Non-ischemic animals are measured to yield >400 x 10⁶ isolated cells per liver. The total cell yield is found to drop rapidly with warm ischemia times of less than one hour to provide 150 to 250 x 10⁶ cells per liver. The total cell yield at times from about 1 hour to five hours is found to be relatively stable at between 50 and 150 x 10⁶ cells per liver. The yield of live cells is decreased rapidly with warm ischemia times of less than one hour and at times of greater than one hour is stable at about 10 x 10⁶ cells per liver. Thus, the proportion of viable cells is found to be bimodal with warm ischemia. At times less than one hour both live and dead cells are precipitously reduced such that the viability ratio is unchanged. At times greater than one hour and up to five hours a stable percentage of viable cells is observed.

The projection areas of liver cell nuclei are measured as a function of warm ischemic time using the above rat model. Livers are perfused, the cells isolated as a single cell suspension, and stained with propidium iodide. Live cells (PI-negative) are collected by flow cytometry, then attached to a glass microscope slide, fixed, permeabilized, and restained with PI to visualize nuclei. Control animals that are perfused without ischemia, are found to have
a bimodal distribution of nucleus areas corresponding to the presence of both mononuclear and binucleated viable liver cells. After one hour and after two hours of warm ischemia, the proportion of binucleated cells is found to decrease. As the binucleated cells are necessarily polyploid, these data are considered to indicate that polyploid cells are more sensitive to ischemia than diploid cells.

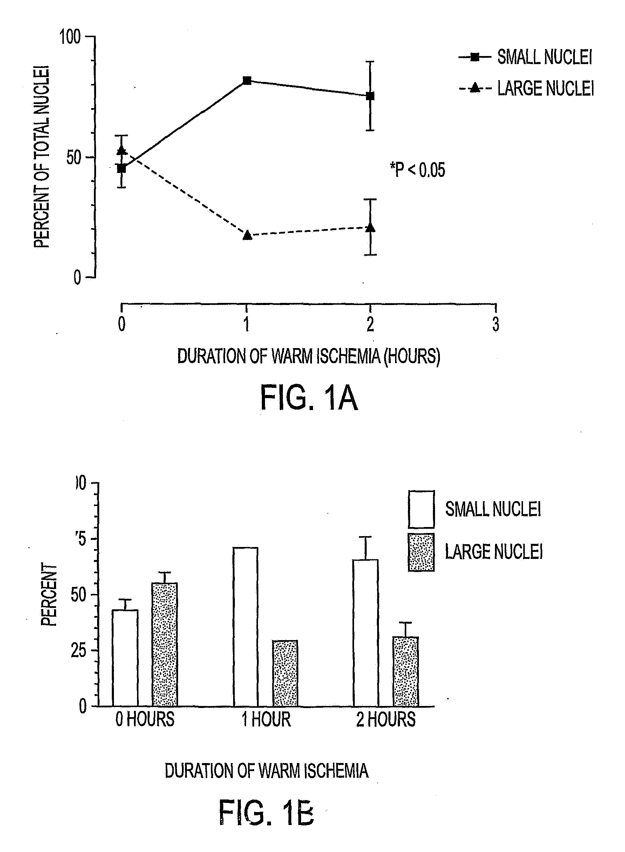

The resistance of diploid cells to ischemia is further supported by analysis of nucleus size in mononuclear cells. Mononuclear cells can be either diploid or polyploid with the diploid cells having smaller nuclei than the polyploid cells. The rat model described above is used to prepare live cells for measurement of the area of nuclei. The percent of total nuclei is presented in figure 1a to indicate that cells with small nuclei are relatively resistant to ischemia. As polyploid nuclei tend to be larger than diploid nuclei, these data are found to indicate that diploid cells are relatively resistant to warm ischemia. The change between nuclear sizes in control livers and livers after two hours of ischemia are found not to differ, as illustrated in figure 1b.

The cell viability is also advantageously examined as a function of time of low temperature ischemia, see Tables 6 and 7. In this embodiment, the liver is rapidly chilled to about 10 °C substantially immediately post-mortem. Even more advantageously, the liver is rapidly chilled to about 4 °C substantially immediately post-mortem. The chilling can be achieved by any of several methods known to those of skill in the art, including, but not limited to the simple expedient of packing the abdomen of the donor cadaver in ice or bags of chilled fluid. The livers are kept at one of the above below-ambient temperatures and are then perfused, as described, at times up to about 30 hours, or more advantageously, at times up to about 20 hours. The single cell suspension resulting from the perfusion is processed to provide progenitors. Polyploid cells are observed to be sensitive to ischemia even if the temperature is maintained below ambient and even at 4 °C.

**Table 6.**

| **Fetal Human Livers** | | |
|---|---|---|
| # | Cold Ischemia (hrs) | Average Viability +/- Std. Dev (Std. Error) |
| 139 | 18 | 75.4±15.9 (1.7) |
| 5 | 66 | 24±8.9 (4) |

**Table 7.**

| **Pediatric and Adult Human Livers** | | | |
|---|---|---|---|
| Fraction | # livers | Cold Ischemia (hrs) | Viability ± Std.Dev (Std. Error) |
| F1 | 9 | <20 | 67.9±18.3 (6.1) |
| F1 | 4 | >20 | 62.5±17.5 (8.8) |
| F2 | 7 | <20 | 83.4 ± 10.3 (3.9) |
| F2 | 6 | >20 | 73 ± 16 (6.5) |
| F3 | 8 | <20 | 81.9 ± 8 (2.8) |
| F3 | 5 | >20 | 75.2 ± 14.5 (6.5) |
| F4 | 16 | <20 | 81.6 ±7.4 (1.9) |
| F4 | 13 | >20 | 21.2 ± 24.9 (6.6) |

Progenitors prepared from donor livers as described in one or more of the above methods are suitable for use in cryopreservation, in flow cytometry, in cell staining, in cell sorting, in liver regeneration, in a bioreactor, in an artificial liver, and as therapeutic treatment, as described below.

### 6.4. Warm Ischemia in a Human Donor

Sample Ren # 200 is received May 21, 1999 from a male, adult donor. The donor is declared brain-dead and evaluated as a donor for organ transplants. However, before the transplant surgeon is able to retrieve the organ, the donor suffers heart arrest. The surgeon is able to remove the liver within 30-60 minutes of heart arrest, that timing constituting the "warm ischemic time". The cross clamp time is 21:19 on May 20^{th}, 1999. The liver is flushed with transport buffer (Viaspan) and put on ice and transported back to UNC. It is received the next morning at 11 AM at UNC (constituting 13 hours and 41 minutes of cold ischemia) and was immediately processed. The processing is found to result in the following cell suspensions with indicated viabilities:

**Table 6**

| *fraction* | *% viability* | *total yield,* | *cells,* |
|---|---|---|---|
| | | *viable cell no.* | *% total* |
| F1 | 69% | 1.5 x 10⁸ | 7.8 |
| F2 | 65% | 3.6 x 10⁸ | 18.8 |
| F3 | 81 % | 5.5 x 10⁸ | 28.8 |
| F4 | 83% | 8.5 x 10⁸ | 44.5 |
| Totals | | 19.1 x 108 | 99.9 |

Thus, processing human liver tissue that was subjected to warm ischemia such as to render it unsuitable for organ transplantation is found to yield isolated cell fractions comprising diploid cells.

### 6.5. Alpha-fetoprotein Expression in Diploid Cells Isolated from Human Liver

In a male patient, age 37, received in the Shock Trauma Unit after a vehicular collision and pronounced DOA, death from asystole is estimated at 25 min. prior to acceptance at the Shock Trauma Unit. The donor corpse is prepared for organ donation by external disinfection. The liver is aseptically removed, packed in an aseptic bag, and chilled for transport to the nearby cell laboratory. Donor liver core temperature is measured by use of a sterile surface temperature probe. A temperature of 10 °C is recorded at 45 min. after estimated time of death. Perfusion of the liver with warm dissociation solution (see above) is begun. A donor liver cell suspension is prepared, as described above, and viable diploid cells isolated by centrifugation on a buffer supplemented with Percoll as above. The isolated cells are divided into aliquots for cryopreservation, for further characterization including antigen typing, and for expansion in cell culture prior to transplantation to an antigen-matched recipient.

In a second male patient, age 34, received in the emergency room after a vehicular accident and pronounced DOA, death from exsanguination resulting from internal lacerations and consequent asystole is estimated at 45 min. prior to acceptance at the Emergency Room. The donor corpse is prepared for organ donation by external disinfection. The liver is aseptically removed, packed in an aseptic bag, and chilled for transport to the adjacent cell laboratory. The donor liver temperature is measured by use of a sterile surface temperature probe. A temperature of 10 °C is recorded at 80 min. after estimated time of death. Perfusion of the liver with solution (see above) is begun. A donor liver cell suspension is prepared, as described above, and viable diploid cells isolated by centrifugation in a buffer supplemented with Percoll as described above. The isolated cells are divided into aliquots for cryopreservation, for further characterization including antigen typing, and for expansion in cell culture prior to transplantation to an antigen-matched recipient.

Samples of the isolated cells from the two donors are prepared for staining with antibody to alpha-fetoprotein, as above, and analyzed by cell sorting on a FACStar cytometer. Cell subfractions corresponding to mononucleated parenchymal cells with small nuclei that are substantially diploid cells are compared to cell subfractions corresponding to polyploid cells, that is, both mononucleated cells with large nuclei and binucleated cells. Comparison of the cells from the age- and sex-matched donors that have experienced different durations of warm ischemia is used to evaluate relative susceptibility of diploid and polyploid liver cells to the effect of warm ischemia. Hepatic progenitors that express alpha-fetoprotein are a subpopulation of the diploid cells of the liver. The ability of cells that express alpha-fetoprotein to survive cold or warm ischemia as equal to or better than that of the other diploid liver cell populations is evaluated.

### 6.6. Development of primers for PCR studies

Analysis of alpha-fetoprotein (AFP) isoforms differentially expressed in hepatic versus other cell types. Cell lines: Two human hepatomas, Hep3B and HepG2, are maintained in Eagle's MEM supplemented with 1 mM sodium pyruvate, 2mM L-glutamine, 50 U/ml penicillin, 50 µg/ml streptomycin, 0.1 mM MEM non-essential amino acid solution, 5 µg/ml insulin and 10% FBS. A human erythroleukemia cell line, K562 and a mouse embryonic fibroblast cell line, STO, are maintained in DMEM/F12 supplemented with 2 mM L-glutamine, 50 U/ml penicillin, 50 µg/ml streptomycin, 5 x 10-5M 2-ME and 10% FBS.

RT-PCR: Total RNAs are extracted from Hep3B, HepG2, and STO by the standard method. The cDNA's are synthesized by oligo-dT priming and subjected to PCR amplification using primer sets designed by the inventors, and prepared for human alpha-fetoprotein (AFP). The primer sequences are as follows,
- hAFP1:: 5'-ACCATGAAGTGGGTGGAATC-3',
- hAFP2:: 5'-CCTGAAGACTGTTCATCTCC-3',
- hAFP3:: 5'-TAAACCCTGGTGTTGGCCAG-3',
- hAFP4:: 5'-ATTTAAACTCCCAAAGCAGCAC-3',
- hAFPexon2:: 5'-CTTCCATATTGGATTCTTACCAATG-3'.
- hAFPexon3:: 5'-GGCTACCATATTTTTTGCCCAG',
- hAFPexon4:: 5'-CTACCTGCCTTTCTGGAAGAAC-3',
- hAFPexon5:: 5'-GAGATAGCAAGAAGGCATCCC-3', and
- hAFPexon6:: 5'-AAAGAATTAAGAGAAAGCAGCTTG-3',
The combinations of the primers are as follows:
hAFP 1 and hAFP2,
hAFP3 and hAFP4,
hAFP1 and hAFP4,
hAFPexon2 and hAFP4,
hAFPexon3 and hAFP4,
hAFPexon4 and hAFP4,
hAFPexon5 and hAFP4, and
hAFPexon6 and hAFP4.

PCR is performed in a total volume of 50µl consisting of 1µM each primer, 200µM each dNTP, 50mM KCI, 1.5mM MgCI2, 10mM Tris HCl, pH8.3, and 1.25U Amplitaq polymerase (Cetus Corp). Samples are heated to 94°C for 3 min followed by amplification for 30 cycles of 2 min at 94°C, 2 min 62°C, and 3 min at 72°C. After the last cycle, a final extension step is performed at 72°C for 7 min. Then 5µl of each PCR reaction is run on 2% agarose gel containing 5 µg/ml ethidium bromide in Tris-acetate-EDTA buffer. Human AFP gene consists of 15 exons. To distinguish truncated transcripts from functional complete AFP mRNA, two different portions of AFP cDNA sequence are selected as target molecules of RT-PCR. The primer combination of hAFP1 and hAFP2 is used for the amplification of exon 1 containing the initiation MET to exon 3, whereas that of hAFP3 and hAFP4 amplify exon 12 to exon 14 containing the stop codon. The results of the PCR are that both combinations of the primers resulted in strongly detected amplification bands in the RNA from Hep3B and HepG2 (lanes 1, 2, 4, and 5). By contrast, only the specific band of the C-terminal portion was detected by the primer set of hAFP3 and hAFP4 in the RNA from K562 (lanes 7 and 8). This result suggests that the erythroleukemia cell line, K562, expresses only a truncated form of AFP without the N-terminus. In support of this hypothesis, the PCR for the whole coding region of AFP using hAFP1 and hAFP4 primers is performed. As expected, the PCR of Hep3B and HepG2 cDNA showed the single remarkable band of 2.1 Kb (lanes 3 and 6), whereas there was no band in K562 (lane 9). The controls are samples with no RNA and a sample derived from the mouse embryonic fibroblast cell line (STO). Neither showed any detectable band. Next, a series of 5' primers from exon 2 to exon 6 are constructed to see the difference between authentic and variant form of hAFP mRNA. The result show that all the cording region except exon 1 is shared in the variant form of hAFP in K562. The combinations of hAFP1 and hAFP4 primers for human AFP RT-PCR that are suitable to detect AFP mRNA expression in hepatic lineages, containing the complete AFP mRNA species. The RT-PCR analysis using this specific combination of primers can eliminate the possibility for any truncated forms expressed in hepatic or non-hepatic cells. This test is used to identify specific subpopulations of liver progenitor cells or to divide hepatic or hematopoietic cell population sharing surface markers.

### 6.7. Processing of donor livers

Cadaveric Livers: Livers obtained postmortem at different times but preferably within at least 24 hours, with a maximum of 30 hours. Livers are processed using a combination of enzymatic digestion and mechanical dissociation, fetal "cadaveric" livers are prepared primarily by mechanical dissociation, whereas the adult cadaveric livers are dissociated primarily by enzymatic digestion. A description of each process is given below. Both fetal and adult livers are digested for varying lengths of time in an enzyme buffer that serves to dissolve the extracellular matrices that bind the cells together in a tissue. The collagenases enzyme mixed used for isolation of liver cells are of high purity "Liberase" enzyme preparation manufactured by Boehringer-Mannheim, consisting of a mixture of purified collagenase and elastase). This enzyme mix is used at much smaller concentrations and with fewer deleterious "side effects."

Enzyme solution: collagenase solution--60-70 mg/100 mls of buffer (Sigma's type IV collagenase, catalog #C5138 or Worthington's type B, catalog # LS005273; both being bacterial preparations enriched in collagenase but with many enzymatic impurities) or Liberase-- (purified collagenase/ elastase preparation by Boehringer-Mannheim, catalog 1814184) prepared in P2 buffer (see below) and used at 0.23 mg/ml

Cell Wash Solution: RPMI 1640 (Gibco) supplemented with insulin (5 µg/ml), transferrin (5 µg/ml), free fatty acid mixture (see below) bound 1:1 molar ratio to purified bovine or human serum albumin.

Free Fatty Acid Mixture: Immature cell populations, and damaged older liver cells, require lipids to maintain and to synthesize their membranes. Although fully mature hepatocytes can synthesize their membranes from a single fatty acid source (linoleic acid) younger parenchymal cells cannot and thus require a mixture of many different fatty acids to handle their lipid requirements. We provide a complex mixture that is then bound in a 1:1 molar ratio with a highly purified albumin bovine serum albumin or highly purified human albumin. In general, human albumin is preferable in order to avoid issues related to "mad cow disease" or bovine spongioform encephalopathy. Accordingly a mixture of free fatty acids is used at a final concentration of about 7.6 µeq /L (7.6 µM) in cell culture media.

The stock solutions are prepared as follows, for a combined total of 100 mM free fatty acids:

| | | | |
|---|---|---|---|
| Palmitic | 31.0 mM | Oleic | 13.4 mM |
| Palmitoleic | 2.8 mM | Linoleic | 35.6 mM |
| Stearic | 11.6 mM | Linolenic | 5.6 mM |

### Preparation of the Individual Fatty Acid Components:

Each individual component is dissolved in 100% EtOH as follows:

| | |
|---|---|
| Palmitic | 1 M stock, soluble in hot EtOH |
| Palmitoleic | 1 M stock, readily soluble in EtOH |
| Stearic | 151 mM stock, soluble in heated EtOH at 1 g / 21ml |
| Oleic | 1 M stock, readily soluble in EtOH |
| Linoleic | 1 M stock, readily soluble in EtOH |
| Linolenic | 1 M stock, readily soluble in EtOH |

These individual stocks are then mixed to obtain the 100mM FFA mixture. Aliquots of the individual FFAs and the FFA mix were made with bubbling nitrogen through to reduce oxidation and increase stability. Stocks are frozen at -20 °C.

P1 Perfusion buffer -- calcium and magnesium free perfusion buffer (pH 7.2) with final concentrations as specified for each of the following components: 118 mM NaCl, 4.7 mM KC1, 1.2 mM KPO4, pH 7.4, 2.5 mM NaHCO3, 0.5 mM EDTA, 5.5 mM glucose, 0.5% bovine serum albumin (BSA), Ascorbic acid (50 µg/ml), Insulin (4 µg/ml), dexamethasone (1 µM).

P2 Perfusion buffer -- Dulbecco's modified Eagle's medium or RPMI 1640 supplemented with 0.5% BSA, Ascorbic acid (50 µg/ml), insulin (4 µg/ml) and dexamethasone (1 µM).

DMEM -- Dulbecco's Modified Eagle's medium (Gibco) with glucose, sodium pyruvate and L-glutamine and further supplemented with 5% fetal bovine serum, insulin (4 µg/ml) and dexamethasone (1 µM). Chee's medium supplemented with ITS+TM culture supplement (5 mls/500 mls) and dexamethasone (0.1 µM). Percoll (Pharmacia) is diluted 9:1 with 10X Dulbecco's phosphate buffered saline.

### 6.8. Cryopreservation experiments

The livers used for cryopreservation methodologies are derived from cadaveric donors as young as fetal livers (gestational ages 12 weeks to 25 weeks) and as old as 77 years of age. A novel cryopreservative buffer is used as follows: Viaspan (Dupont Catalog # 1000-46-06) supplemented with 2% human serum (Gibco) or fetal bovine serum (Biowhittaker), 10% cryopreservative dimethylsulfoxide (Sigma catalog #D5879 or D8779) used exclusively for mature parenchymal cells or dimethyl sulfoxide or glycerol (Sigma catalog # G6279) used for progenitors]. The buffer is further supplemented with antibiotics (penicillin at 200 U/ml; streptomycin at 100 µg/ml). The buffer is further supplemented with hormones and growth factors: insulin (5 µg/ml), transferrin (5 µg/ml), epidermal growth factor (50 µg/ml), FGF (10 ng/ml), IGF II (10 ng/ml). The buffer is further supplemented with lipids: free fatty acids (7.6 µM) bound to bovine serum albumin (BSA) or human serum albumin (HSA) and high density lipoprotein (10 µg/ml) The buffer is further supplemented with trace elements (selenium (10⁻⁹M), copper (10⁻⁷M), zinc (5 X 10⁻¹¹ M) and an antioxidant, AEOL 10112 (a proprietary antioxidant, a porphorin that is a superoxide dismutase mimetic used at 10 µg/ml), a product prepared by AEOLUS, a subsidiary of Incara.

The variation in the composition, as disclosed herein, is to combine the key nutrients, lipids, hormones and growth factors that are identified as part of serum-free hormonally defined media tailored for liver cells. The novel buffer results in viabilities of the liver cells for the F4 fractions that are as low as approximately 10% or less (from very poor samples collected at the upper limit of time of about 30 hours postmortem) to as high as 80% (for good samples collected at early time periods closer to one hour or above). The viabilities of the F1-F3 fractions are consistently above 40%, a fact attributed these fractions being "younger" cells with ploidy states and metabolic activity more conducive to synthesis of extracellular matrix components and/or other cellular factors needed for viability and growth; thus, they are likely to be easier to freeze. The use of superoxide dismutase mimetic in the buffer increased the viability of the cells by 5-10%.

An alternative to the above is to use a modified buffer in which the Viaspan is eliminated and the basal medium (such as RPMI 1640) is supplemented with insulin (5 µg/ml), transferrin (5 µg/ml), free fatty acids (7.6 µM) bound to BSA, high density lipoprotein (10 µg/ml), trace elements (selenium (10⁻⁹M), copper (10⁻⁷M), zinc (5 X 10⁻¹¹ M)), and AEOL 10112. Coat the cells with a form of extracellular matrix such as type IV collagen mixed with laminin or type III collagen mixed with fibronectin.

Fetal "cadaveric" liver cells, processed as described above, are suspended in the cryopreservation buffer (described above), aliquoted into 3 ml cryovials at 5-10 X 10⁶ cells/ml and maintained under that condition for 1-2 hours. The cells are then frozen to liquid nitrogen temperatures of -160°C using a computerized control rate freezer (Forma Cryomed) and then stored in a large vapor phase, liquid nitrogen (-160°C) storage tank. Cells survive the process well and with no significant loss of viability occurs over storage periods ranging from 50-270 days (see Figure 4).

The extreme range of viabilities of the F4 fractions both after processing and after freezing are due to the varying lengths of time between "clamp time" and receiving the samples in the lab and also to the varying conditions of the liver (fibrotic, ischemic, etc.). In general, the F4 fraction is the most sensitive to the vagaries of treatment of the livers and the general health of the tissue. Remarkably, the F2 and F3 fractions were routinely viable and readily cryopreserved even when obtained from poor liver specimens. The F1 fractions were more variable, containing a large amount of debris, fat droplets as well as numerous small cells that included both small parenchymal cells (assumed to include hepatic progenitors) and various hemopoietic subpopulations (i.e., erythrocytes).

**Table 7. Cryopreservation: Fetal Liver**

| | |
|---|---|
| Average viability after processing: | 75-85% |
| Average viability after processing: | equivalent to that after processing |

**Table 8. Cryopreservation: Adult Liver**

| | | |
|---|---|---|
| Viability (after freezing) | | |
| | F1-F3: | >75% with good attachment |
| | F4: | <60% with poor attachment |

### 6.9. Flow cytometry

The following sorting method is optional. The cells are passed in single file through a flow cell where they are exposed to laser light. The approximate volume of each cell is determined by "forward scatter", or the amount of light that is refracted as the beam is intersected. Scattered light, "side scatter" from internal cellular structures such as the nucleus, endoplasmic reticulum Golgi bodies, vesicles, etc., are used to determine the amount of internal complexity (i.e. an active cell and a more mature cells will contain more internal components than a quiescent one or a younger one). More selective information on cell characteristics is obtained by binding highly specific, characteristic antigens to protein complexes on the cell surface. These antibodies can be covalently bonded to fluorescent molecules such as Fluorescein Isothiocyanate (FITC), Phycoerythrin (PE), and tandem conjugates of PE and Cytochrome which are excited by the laser beams, generating emitted light at specific wavelengths for each fluorophore. By selecting a panel of distinctive chromophores conjugated to specific antibodies cell populations of interest are selected.

Cells were analyzed based on their parameters input. A variety of collection devices are used to collect the desired cells, including Eppendorf and conical tubes, and any size multi-well plate at the speed of up to 40,000 events per second or higher.

**TABLE 9. Antibodies and reagents used in staining procedures**

| *Antibody* | *Supplier, Cat #,* | *Lot #* |
|---|---|---|
| Goat anti-human AFP | Chemicon, | AB635, C4P168 |
| Monoclonal mouse X human Thy | Chemicon, | MAB 1294, 293CCD |
| Monoclonal mouse antihuman | | |
| AFP-PE conjugate | Chromaprobe, | P41020, A45P7 |
| Biotinylated Rabbit anti-Goat | Vector Laboratories, | BA-5000,J0313 |
| Biotinylated Rabbit anti-Goat, | Jackson Immunochemicals | 200-152-096,25985 |
| Streptavidin/AMCA conjugate, | Jackson Immunochemicals, | 016-150-084,40001 |
| Donkey anti-sheepAMCA conjugate, | Jackson Immunochemicals, | 713-156-4732202 |
| Donkey anti-Goat CY5 conjugate, | Jackson Immunochemicals, | 705-156-147,38756 |
| Goat IgG, | Jackson Immunochemicals, | 005-000-002, 38837 |
| Sheep IgG | Jackson Immunochemicals, | ,013-000-002, 39945 |
| Sheep anti-human Albumin, | Serotec, | ABP102,210498 |
| Mouse monoclonal anti-human: | | |
| CD14/Tri Color conjugate | Pharmingen | |
| ICAM | Pharmingen | |
| CD34/FITC conjugate | Pharmingen | 34374X |
| CD38/PE conjugate | Pharmingen | 31015X |
| CD38/FITC conjugate | Pharmingen | 31014X |
| Glycophorin A PE conjugate | Pharmingen | 32591A |
| CD 45/PE conjugate | Pharmingen | 31255X |
| CD 45/FITC conjugate | Pharmingen | 31254X |
| Isotype controls IgG1 PE | Pharmingen | 33815X |
| IgG2 FITC | Pharmingen | 33814X |
| Kit PE conjugate | Caltag | MHCK04 |
| Rabbit X Human AFP-FITC conjugate | Accurate | |
| Goat anti-Human AFP unconjugated | " | AXL625 061 |
| 7Amino Actinomycin D (7AAD) | Mol Probes | A-1310,4981-1 |

Principal solutions used in cell preparations for flow cytometry:
BSA: bovine serum albumin (Pentex V)
PBS = phosphate buffered saline;
FBS = fetal bovine serum;
AFP = alpha-fetoprotein

Dulbecco's Modified Eagles Medium with Hormones: HC_DMEM
500 mL DMEM, high glucose without phenol red
25 mL fetal bovine serum (FBS)
20 mL 5mM EGTA
Insulin (5 µg/ml), transferrin (5µg/ml)
Trace elements [selenium (10⁻⁹M), copper (10⁻⁷M), zinc (5 X 10⁻¹¹ M)]
Antibiotics (Penicillin-100 µg/ml, streptomycin-100 µg/ml)
500 mg bovine serum albumin (BSA) 30 mg DNase
38µl free fatty acid solution bound to BSA.
Sterile filtered through a Nalgene filtration unit with 0.2 µm pores

Hanks Buffered Saline Solution-modified version: HBSS-mod
50 mL 10X HBSS
10 mL 1MHepes
Penicillin-100 µg/ml/Streptomycin-100 µg/ml
500mg BSA
30 mg DNase
Make up to 400 mL
pH to 7.3
Top up to 500 mL
Sterile Filter at 0.2 µm

Blocking buffer for immunochemistry
100 mls of HBSS_mod
2.2 mL 45% teleostean fish gel and
0.8g BSA
0.5mL 1% saponin in HBSS

Mounting medium for Immunofluorescence microscopy
0.5 mL 2X PBS
0.25g n-propyl gallate
5.7g glycerol

### 6.10. Procedures for preparation of frozen liver tissue for flow cytometry

Thaw frozen liver tissue rapidly at 37°C. Each cryovial of liver (each containing about 3 mL of buffer containing 5-10 X 10⁶ cells/mL) is brought up to 10 mL at a rate of 1 mL per min. on ice with HC-DMEM. The sample is then centrifuged at 1200 RPM for 5 min at 4°C. The supernatant is discarded, and the pellet of cells resuspended in 5 mL of HC-DMEM. The washing of the cells is repeated until the supernatant becomes clear. Then the cells are counted and the viabilities assessed with a hemocytometer using the trypan blue dye exclusion assay. The cells are split into fractions according to the experimental protocol. Standard tubes are prepared for control data containing between 1 and 2 X 10⁶ cells, usually achieved by taking 200 µl for each from a cell suspension of 5-10 X 10⁶/mL. The following standard tubes are needed:
1) OCS. Original cell suspension which consists of unstained control cells.
2) FITC alone for compensation adjustments. Add 5 µL of FITC-labeled anti glycophorin A to 200 µl of cell suspension. Alternative is a cocktail of FITC-labeled CD34, CD38 and CD45, 7 µl of each into 200 µl of cells.
3) PE alone for compensation adjustments. Use a Glycophorin-PE (2 µl to 1 mL HC_DMEM and add 30 µL of this to 200 µL of cells).
4) 7AAD alone for compensation. A good signal is generated by fixing 200 µL of cell suspension with 2% paraformaldehyde and then adding 5 µL of 100 µM 7AAD and 5 µL of detergent (1% saponin) to a 1 mL suspension of these cells in HBSS-mod. The permeabilized cells stain intensely with 7AAD.
5) Cy5 alone for compensation 200 µL of fixed cells (2% paraformaldehyde) are incubated for 40 min in 2% goat serum to label the cell surfaces with sheep IgG. The cells are then incubated with Cy5 conjugated donkey anti-goat IgG (1: 800) for 40 min.
6) AMCA alone for compensation. As with 7AAD, an artificially intense signal is generated for compensation adjustments. 200 µL of fixed cells (2% paraformaldehyde) are incubated for 40 min in 2% sheep serum to label the cell surfaces with sheep IgG. The cells are then incubated with AMCA conjugated donkey anti-sheep IgG (1: 800) for 90 min.
7) AMCA/Cy5 controls. Incubate fixed (2% paraformaldehyde) and permeabilized (0.05% saponin) cells with AMCA-conjugated donkey anti sheep IgG and Cy5-conjugated donkey anti goat IgG for 90 min.
8) Monoclonal Isotype controls. Incubate cells with a mouse IgG1 PE conjugate and a mouse IgG2 FITC conjugate. Concentrations should match those used to label analytical and sort tubes.
9) Intracellular Isotype Controls. Incubate fixed (2% paraformaldehyde) and permeabilized (0.05% saponin) cells with non-immune sheep IgG and goat IgG for 90 min as controls for antibodies used for identification of albumin and alpha-fetoprotein. Continue with incubation with Cy5-conjugated donkey anti-goat IgG and AMCAconjugated donkey anti sheep IgG for 90 min.

Sort tubes are prepared for the acquisition of selected cell populations expressing particular combinations of CD markers. Normally these tubes contain 50-70 X 106 cells. Cells are resuspended in 1 mL of staining buffer comprised of HC_DMEM + 1% BSA + 500 pM 7AAD (5 µL of 100 µM stock). Between 15 and 25 µL each of CD 34 FITC, CD38 PE, or CD 45 PE are added to the staining buffer according to cell numbers (normally 3 µL of Pharmingen antibody per 10 X 10⁶ cells). Antibody to c-Kit is added at a 1:60 dilution, glycophorin A is used at a 1:500 dilution. Stain for 40 min on ice in the dark. After staining wash cells twice with HBSS-mod and fix with 2% paraformaldehyde in PBS for 30 min on ice.

### 6.11. Intracellular staining for cell sorting

For intracellular staining of cells for analysis of alpha-fetoprotein (AFP) by flow cytometry the cell suspension is permeabilized with a solution of saponin (Sigma S4521) 0.05% in HBSS_ mod for 10 min on ice: Cells are then blocked in a solution of HBSS_mod containing 1% teleostean fish gel and 0.8% BS and 0.005% saponin for 20 min, followed by incubation with goat anti-human AFP and sheep anti human albumin (both 1:800 in blocking buffer) for 90 min at room temperature in the dark. Cells are washed twice with HBSS_mod containing 0.01% saponin followed by incubation with Cy5-conjugated donkey anti-goat IgG and AMCA-conjugated donkey anti sheep IgG for 90 min.

Alternatively, following the primary antibody, cells are incubated with biotinylated rabbit anti goat IgG (1: 500 in blocking buffer containing 2% human serum and 0.01% saponin for 90 min at room temp in dark). This is followed by 2 washes with HBSS_mod containing 0.01% saponin and then incubation with 9 µg/mL streptavidin/Cy5 conjugate in 0.01% saponin/ HBSS-mod for 90 minutes at room temperature in dark. Finally, cells are washed 2 times with HBSS-mod and resuspended in HBSS-mod, filtered though a 50 µm sieve to remove clumps of cells for analysis and sorting on the flow cytometer.

If selection of hepatic progenitors is intended, the immunoselection includes removing cells that are polyploid and/or express markers associated with mature hemopoietic cells from the liver such as glycophorin A on red blood cells. Additionally cells exhibiting CD45, which is expressed on all mature hemopoietic cells; cells exhibiting markers associated with mature hepatic cells such as connexin 32, which is found on all hepatocytes and biliary cells; and cells expressing markers associated with mature mesenchymal cells, such as retinoids in hepatic stellate cells or von Willebrand Factor or Factor 8 in endothelia, are all removed.

### 6.12. Immunohistochemical staining of sorted cell populations

Cells are stained for alpha-fetoprotein after analysis and sorting by the flow cytometer. The sorted cell fractions are collected in 0.3% HBSS-mod containing 1% BSA. Upon return to the laboratory the volume of collected samples is adjusted to provide 0.5 X10⁶ cells/mL and 200 µL aliquots are spun onto microscope slides with a Shandon Cytospin apparatus. The cytospun slide preparations are air dried and stored for later staining for alpha-fetoprotein and/or albumin. The attached cell "disk" of the microscope slide are ringed with a rubber dam to produce a "well" for application of immunohistochemical reagents. Slides are soaked in tris buffer ("low salt" 10 mM Tris with 0.9% NaCl at pH 7.4) containing 0.3% Triton X for 10 min, followed by 10 min in low salt Tris alone.

Cells are then blocked in 10% rabbit serum contained in a teleostean gel blocking solution described above for 90 min at room temperature. After two washes in low salt Tris cells are incubated overnight at 4 degrees C with goat anti-human AFP antibody diluted to 1:100 in blocking buffer containing 2% rabbit serum. Two washes in Tris buffer are then followed by a 90 min incubation with biotinylated rabbit anti goat IgG (1:200) in blocking buffer at room temp. Final incubation with streptavidin/AMCA complex (9 µg/mL in low salt Tris buffer) is used to locate AFP-like immunoreactivity through binding of the AMCA fluorochrome with the biotinylated rabbit antibody. Following 2 washes with Tris buffer the cell preparations are allowed to come close to dryness before coverslipping under an antifade mounting medium (0.25g n-propyl gallate in 5:7g glycerol with 1 mL PBS). When appropriate cells are double-stained for albumin by including a Texas red conjugated rabbit anti human antibody against albumin with the primary anti-fetoprotein antibody.

Control slides are prepared by omission of the primary or the secondary antibody to demonstrate no AMCA labeling of cells in the absence of either the anti alpha protein antibody or the biotinylated secondary antibody. Slides are inspected with epifluorescence microscopy using UV excitation of the AMCA dye which emits light in the blue (450 nm) region.

### 6.13. Liver regeneration by means of cell and/or gene therapy

This invention has immediate practical application to treat diseases such as Crigler-Najjar syndrome, Dubin-Johnson syndrome, tyrosinanemia, cirrhosis, fibrosis, fatty liver, hepatitis, acute liver failure, chronic liver failure, hepatocholangitis, hepatomalacia, hepatomegalia, hepatocarcinoma, hepatoblastoma, or combination thereof. Other liver diseases of this example and other relevant examples of liver diseases are equally eligible as candidates for the instant therapy and include Alagille syndrome, alcoholic liver disease, alpha-1-antitrypsin deficiency, autoimmune hepatitis, Budd-Chiari syndrome, biliary atresia, Byler disease, cancers such as extrahepatic bile duct carcinoma and hepatocellular carcinoma, Caroli disease, galactosemia, Gilbert syndrome, glycogen storage disease i, hemangioma, hemochromatosis, hepatitis A, hepatitis B, hepatitis C, hepatitis E, hepatitis G, liver transplantation, porphyria cutanea tarda, primary biliary cirrhosis, protoporphyria, erythrohepatic, Rotor syndrome, sclerosing cholangitis, and Wilson disease. Inborn genetic diseases of the liver are also correctable as well. For example, genetic disease phenylketonuria (PKU) is caused by a baby's inability to use the amino acid phenylalanine. If not treated early, PKU leads to brain and nerve damage and mental retardation. A special low-protein diet beginning in the first weeks of life is the only available treatment at present time. Examples of other target genes and their related liver diseases that are amenable to this form of therapy include, but are not limited to, the LDL receptor gene in familial hypercholesterolemia, the clotting factor genes for factors VIII and IX in hemophilia, the alpha-1-antitrypsin gene in emphysema, the phenylalanine hydroxylase gene in phenylketonuria, the ornithine transcarbamylase gene in hyperammonemia, and complement protein genes in various forms of complement deficiencies.

Since human urokinase plasminogen activator (uPA) can activate plasminogen across species a recombinant adenoviral vector that expresses human urokinase from the RSV-LTR promoter, Ad-RSV-uPA is constructed with the aim to induce liver regeneration. This gene is selected only by way of illustration as any other genes of interest are equally suitable including but limited to carbamoyl synthetase I, ornithine transcarbamylase, arginosuccinate synthetase, arginosuccinate lyase, arginase, fumarylacetoacetate hydrolase, phenylalanine hydroxylase, alpha-1 antitrypsin, glucose-6-phosphatase, low-density-lipoprotein receptor, porphobilinogen deaminase, factor VIII, factor IX, cystathione beta.-synthase, branched chain ketoacid decarboxylase, albumin, isovaleryl-CoA dehydrogenase, propionyl CoA carboxylase, methyl malonyl CoA mutase, glutaryl CoA dehydrogenase, insulin, transferrin, beta-glucosidase, pyruvate carboxylase, hepatic phosphorylase, phosphorylase kinase, glycine decarboxylase, H-protein, T-protein, Menkes disease protein, the product of Wilson's disease gene pWD, and/or CFTR.

For construction and production of the recombinant adenoviral vectors, the cDNA for human uPA is prepared as follows. The 1.326 kb HindIII/Asp718 uPA fragment that contains the protein coding sequence is inserted into the HindIII/Asp718 sites of pXCJL.1 under the transcriptional control of the Rous Sarcoma Virus LTR (RSV) promoter, and upstream of the bovine growth hormone polyadenylation signal. One skilled in the art can select liver cell-specific promoter such as hepatitis B promoters, hepatitis A promoters, hepatitis C promoters, albumin promoters, alpha-1-antitrypsin promoters, pyruvate kinase promoters, phosphoenol pyruvate carboxykinase promoters, transferrin promoters, transthyretin promoters, alpha-fetoprotein promoters, alpha-fibrinogen promoters, and beta-fibrinogen promoters among many other suitable promoters.

The virus is prepared after co-transfection with pJMI7 and the vector designated Ad-RSV-uPA. The screening for Ad-RSV-uPA is carried out by amplification of individual plaques in 293 cells. Three days after infection the supernatant is tested for immunological reactive uPA by ELISA and fibrinolytic activity by fibrin plaque assay demonstrating the catalytic activity of uPA produced upon Ad-RSVuPA infection. The purified virus is stored in aliquots at -80 °C and freshly diluted with HgDMEM media prior to injection. The viral titers are determined by OD measurements and standard plaque assay. The construction of the vectors is essentially carried out as described in the U. S. Pat. No. 5,980,886, incorporated herein by way of reference. The viruses are titered on 208F cells.

C57BL/6 female mice aged 5 to 6 weeks (Jackson Laboratories, Bar Harbor, ME) are housed in a specific pathogen free environment. Ischemic liver samples at various time periods are obtained from euthanased mice and liver progenitors are isolated as disclosed supra. For portal vein cannulation, recipient mice are anesthetized by an intraperitoneal administration of 0.5 ml of 20 mg/ml 2,2,2-tribromoethanol. A midline abdominal incision is made and the skin is separated from the peritoneum to create a subcutaneous pocket. The peritoneum is opened and the portal vein is exposed. A silicone tube (0.02" I.D., 0.037" O.D., S/P Medical Grade, Baxter, Ill.) is inserted in the portal vein and perfused with heparinized saline. Thereafter the cannula is tunneled through the peritoneum and secured with a 4.0 silk suture. The 3cm long cannula is tied off at the distal end and placed subcutaneously in the previously created pocket. The mice are given the virus-infected progenitor cells no earlier than 24 hrs later. In some mice the portal vein cannulation is performed together with a 2/3 hepatectomy. The partial hepatectomy is then carried out. To perfuse the portal vein, mice are anesthetized, the skin is opened at the proximal site of the already existing abdominal incision. The cannula is exposed and connected to a syringe pump. For virus infusion, the preps of adenovirus in DMEM are injected over 5 to 10 min into the portal vein through the cannula. For the purposes of cell therapy any cell populations are used as an autologous or allogenic material and transplanted to, or in the vicinity of, a specific target organ of a patient such as a case in this example. Cells can be transplanted in any suitable media, carrier or diluents, or any type of drug delivery systems including, microcarriers, beads, microsomes, microspheres, vesicles and so on.

All biochemical and histological analysis are performed after injection of adenovirus-infected hepatic progenitors into the portal vein through the cannula. The ELISA assay for uPA is based on two different monoclonal antibodies directed against the catalytic and receptor-binding domain of uPA. One of the monoclonal antibodies is labeled with peroxidase. Serum total protein and albumin are analyzed by routine automated methods in the clinical pathology laboratories. Infusion of adenovirus into the portal vein of C57BI/6 mice is known to result in transduction of 100% of hepatocytes with more than 1 copy of adenoviral DNA per cell. The same dose of Ad-RSV-uPA results in 90% mortality that at least in part was related to hemorrhage. When lower dose of Ad-RSV-uPA is used, the mortality rate is less than 5% and this dose is selected for the liver regeneration experiments. The infusion of Ad-RSV-uPA results in transient elevations of serum urokinase reaching a peak value of about 350 ng/ml (70 to 100 times greater than endogenous levels) four days later before falling to background concentrations by day 12. The rise in uPA is also associated with an increase in the serum SGPT concentrations. At varying times after adenovirus infusion, animals are infused with ³H-thymidine, and the amount of radioactivity incorporated into liver DNA is determined as a means to quantitate cell proliferation. The animals treated with Ad-RSV-uPA had an increased period of thymidine uptake that began on day 3 and persisted for 8 days. Thus, the period of hepatic 3H-thymidine uptake with Ad-RSV-uPA/oval cells treatment is much greater than that obtained with partial hepatectomy alone. The recipients of the negative control adenovirus show peak of hepatic 3H-thymidine uptake on day 4 that returned to baseline levels 24 h later and a minimal rise in 3H-thymidine uptake on day 11. In summary, the hepatic reaction as measured by SGPT levels and high rates of 3H-thymidine uptake is attributed to intrahepatic urokinase production indicating that significant liver biosynthetic regeneration occurs. Hepatic progenitor cells infused without uPA are better than adenovirus without uPA insert.

Microscopic histological findings from animals treated with recombinant adenovirus/progenitors derived from non-heart beating cadaver donors indicate that by day 3 treated mice have a moderate inflammatory infiltrate that contains macrophages and neutrophils. Degenerative changes in hepatocytes include vacuolization, pyknotic and few mitotic nuclei. Eight to 10 days after Ad-RSV-uPA/oval cell administration there is evidence of hepatic recovery including the presence of multifocal regeneration, heterogenous size of nuclei, and a much decreased inflammatory reaction with few degenerating hepatocytes. By three to four weeks, the infiltrate resolves and the liver appears normal.

In total, these studies demonstrate that urokinase expression in combination with hepatic progenitors induced significant liver parenchymal cell regeneration.

### 6.14. Bioreactor

A high performance bioreactor (HPBR) is employed to cultivate human hepatocyte progenitors isolated from a cadaver donor. This process will provide a large number of cells useful for further medical purposes or bioreactor by itself serves as a production unit for biologically useful cell-secreted proteins and factors that can include, but are not limited to hepatocyte growth factor (HGF), insulin-like growth factor-I and II (IGF-I and II), epidermal growth factor (EGF), type a and type b transforming growth factor (TGF-alpha and TGF-beta), nerve growth factor (NGF), fibroblast growth factor (FGF), platelet-derived growth factor (PDGF), sarcoma growth factor (SGF), granulocyte macrophage colony stimulating growth factor (GM-CSF), vascular endothelial growth factor (VEGF), prolactin and growth hormone releasing factor (GHRF) and various hemopoietic growth factors such as interleukins (IL) IL-1, IL-2, IL-3, IL-4, IL-5, IL-6, IL-7, IL-8, IL-10, IL-11, etc., erythroid differentiation factor (EDF) or follicle-stimulating hormone releasing protein (FRP), inhibin, stem cell proliferation factor (SCPF) and active fragments, subunits, derivatives and combinations of these proteins among many others known in the art. Generally, as used herein, these cellular factors refer to a secreted protein which is selected from the group consisting of a cytokine, a lymphokine, an interieukin, a colony-stimuiting ractor, a hormone, a chemotactic factor, an anti-chemotactic factor, a coagulation factor, a thrombolytic protein, a complement protein, an enzyme, an immunoglobulin, and an antigen. Among such biologically active proteins one skilled in the art can select Factor VIII, Factor IX, Factor VII, erythropoietin, alpha-1-antitrypsin, calcitonin, growth hormone, insulin, low density lipoprotein, apolipoprotein E, IL-2 receptor and its antagonists, superoxide dismutase, immune response modifiers, parathyroid hormone, the interferons (IFN alpha, beta, or gamma), nerve growth factors, glucocerebrosidase, colony stimulating factor, interleukins (IL) 1 to 15, granulocyte colony stimulating factor (G-CSF), granulocyte, macrophage-colony stimulating factor (GM-CSF), macrophage-colony stimulating factor (M-CSF), fibroblast growth factor (FGF), platelet-derived growth factor (PDGF), adenosine deaminase, insulin-like growth factors (IGF-1 and IGF-2), megakaryocyte promoting ligand (MPL), thrombopoietin, or combinations thereof.

Without limiting to this particular protocol of growing cells in a bioreactor, other well-known in the art procedures are equally suitable and can be easily adopted from published U.S. Pat. Nos. 6,001,585; 5,998,184; 5,846,817; 5,622,857; 5,571,720; 5,563,068; 5,512,474; 5,443,985; 5,342,781; 5,330,915; 5,320,963; 5,202,254; 4,833,083; and 4,760,028 as incorporated herein by way of reference.

The instant device contains 450 10 kD cellulose fibers 540 polypropylene fibers and details on other parameters are found for example in U. S. Pat. No. 5,622,857 as incorporated herein by way of reference. Cells are isolated as disclosed above. All necessary materials are obtained from either Sigma Chemical Co. or Life Technologies. Attachment media for long-term culture media is as follows: RPMI 1640 (500 mL); 50 mL (10%) FBS; 4 mM L-glutamine; 1x Penicillin/streptomycin; Gentamicin; 15 mM HEPES; 10 mU/mL Insulin; 10 mU/mL Transferrin; Selenium; The HPBr system is flushed with media for one day before attachment media is applied. 500 mg of preswollen Cytodex 3 microcarriers are inoculated in the inner annular space of the HPBr. The oxygenator fibers cradled the microcarriers and prevented them from distributing throughout the ECS. Viable human hepatocyte progenitors are also inoculated into the inner annular space, and the device rocked and rotated by hand to achieve uniform mixing of cells and microcarriers. Assuming that the hepatocytes are between 10-20 µm diameter, the cell-to-microcarrier inoculum ratio is about 500. The apparent viscosity of cells and microcarriers increases rapidly, indicating that cell-to-microcarrier and cell-to-cell attachments are proceeding rapidly and normally. Within a 2-3 minutes of this mixing a discrete gel of cells and microcarriers is formed in the inner annular space. Following an overnight incubation at 37 °C in attachment media (in a stationary position), the media is changed to long-term culture media (2 L). These volumes are not limiting in any way as one skilled in the art can scale easily the production to the desired level. The hepatocytes are cultured for 5 weeks, with fresh media applied to the system weekly. The metabolic function of the cells is monitored by testing daily samples. After 5 weeks, >90% recovery of viable cells and microcarriers is achieved by the following procedure: 0.1% collagenase in PBS mixed with 0.44 mL (0.23 M) EDTA is used to flush the ECS and the HPBr incubated for 10 minutes; the content of the ECS is expelled with sterile air from a syringe barrel; this process is repeated with long-term culture media and the materials collected washed and separated.

The HPBr is equally suitable in the cultivation and genetic transformation of cells (e.g., HGF gene expression). The following is a genetic non-viral protocol for anchorage dependent cells (e.g., SW 480 P3; ATCC #CCL228), that can be appropriately modified and optimized from published procedures using culture wells and dishes, by those skilled in the art. Media fiber with 10 kD properties are preferred in the HPBr. The bioreactor is operated in much the same manner as described supra. Cytodex 1 microcarrier (Pharmacia, sold by Sigma Chemical Co.) are widely use for culturing anchorage dependent cells. A broad range of cell densities can be inoculated into the ECS of the HPBr, ranging from: 1x10⁴ to 1x10¹⁵ cells or higher as desired. The recommended cell-to-microcarrier inoculum ratio is in the range of about 10, although one skilled in the art can modify this as desired. The device is gently rotated throughout the experiment at about 10 cpm (or greater). After culturing the cells for about one day (or more, depending on the specific cell), optimal confluence is attained to obtain efficient transfection. The cell-to-microcarrier inoculation ratio is adjustable to positively impact this time frame for therapeutic and economic efficiency. On the day of the transfection, prepare the DNA plasmid solution (e.g., pCMV), and cationic lipid solution (e.g., LIPOFECTIN Reagent, Life Technologies). These reagents most be serum free, even if the overall process requires the presence of serum. Mix appropriate quantities of DNA and lipid solutions, then inject the mixture into the ECS of the device. After about a few (or even several) hours of transfection, resume use of serum, if appropriate, and continue to culture cells as before for about a few days. Longer periods can be used when expanding permanently transformed cells. Harvest cells in a manner similar to that described previously.

### 6.15. Artificialliver

As an extension of above example one skilled in the art can easily adopt the bioreactor as an extracorporeal hepatic support system. Xenotransplantation (the transplantation of organs between species) can help alleviate the shortage of donor livers by using animal organs. A potential danger of transplanting animal organs into humans, however, is that viruses that infect the donor animals can infect the recipients. As the organ transplant recipients would be taking drugs to suppress the immune system and prevent organ rejection, they can be unable to fight off the infecting animal virus. Alternatively, the animal virus can mutate in the infected host into a form that can infect human contacts with normal immune systems. As a result, a new pathogenic human virus can arise. An extracorporeal hepatic support system overcomes these drawbacks. Favorite animal species for human organ transplantation are the pig and primates. Nevertheless it is clear that if a human cell-based artificial liver is available, it is preferable to animal livers.

After desired time in culture matured hepatocytes derived from cadaveric liver progenitors are obtained. Routinely 2 to 5 billion cells of high (over 80%) viability are obtained. In general the culture medium used is the hormone-supplemented Waymouth medium. To accommodate 2 to 5 billion cells, the bioreactor is scaled up to two containment vessels, each with an internal diameter of 40 mm and a height of 100 mm. In this particular situation glass beads of approximately 2 mm in diameter and a total volume of 250 ml per containment vessel are used. Medium is supplied at a recycle rate of 360 ml/min. The high viability of the hepatocytes is evidenced by the stable oxygen consumption rate. The bioreactor is then attached to an ahepatic human recepient whose liver is removed by surgery due to total hepatic failure. Alternatively, the liver is not removed but instant bioreactor will help the better recovery of dysfunctional liver.

A skilled artisan will know the procedures for attaching of the bioreactor as an extracorporeal hepatic support system or will know alternative means known in the art such as disclosed for example in the U. S. Pat. Nos. 6,008,049; 5,981,211; 5,976,870; 5,891,713; 5,827,729; 5,643,794; 5,622,857; 5,605,835; and 5,270,192, each incorporated herein by way of reference in its respective entirety. It is evident from such references that donor artificial liver cells are not necessary limited to human species and cross-species use of such cells is now possible. For example, liver cells from pigs or primates are equally suitable for human use.

Blood from the left femoral artery is directed into a Minntech hemoconcentrator. A 12 fringe elecath cannula is inserted into the femoral artery and connected to a 1/4" PVC tubing to the hemoconcentrator. The hemoconcentrator separated the blood into a cell free ultrafiltrate fraction, and a blood cell fraction. The blood cell fraction is returned to the femoral vein via a similar tubing. The ultrafiltrate exited the hemoconcentrator via a 1/4" PVC tubing and entered the hepatocyte bioreactor system with the flow rate adjusted to 40 ml/min. using a roller pump. After perfusion through the bioreactor, the ultrafiltrate is returned to the patient via the left jugular vein. To demonstrate the provision of extracorporeal hepatic metabolism, two different chemicals known to be metabolized by the liver, 7-ethoxycoumarin and lidocaine, are administered into the ultrafiltrate at the inlet of the bioreactor. The respective metabolites, 7-OH-coumarin and monoethylglycinexylidide (MEGX), are measured at the outlets of the bioreactors before the ultrafiltrate is returned to the patient. Significant metabolism of both 7-ethoxycoumarin and lidocaine are observed. The results therefore demonstrate the application of the bioreactor as a support system, providing extracorporeal hepatic metabolism. The separation of the blood cells from the plasma minimizes immunological reaction of the recipient to the foreign hepatocytes. Hepatocytes from human donors as in our example liver cells obtained from cadavers and nonhuman, source such as pig, are thus useful in the bioreactor to provide extracorporeal hepatic support.

### 6.16. Progenitor cadaveric cells other than liver cells

This invention also relates to methods of obtaining cell populations enriched in progenitor cells from tissues other than liver. Examples of such tissues include but are not limited to adrenal gland, blood vessel, bone marrow, cornea, islets of Langerhans, bile duct, lens, lung, kidney, heart, gut, ovary, pancreas, parathyroid, pineal, pituitary, skin, testis, bladder, brain, spinal cord, thymus, or thyroid.

The following examples are provided as a general strategy that can be modified according to particular needs but without altering the scope and spirit of the invention. In an exemplary embodiment, the subject progenitor cells are provided which are useful for patients suffering from any insulin-deficiency disorder.

Both fetal and non-fetal cadavers are used in these studies. After exsanguination, the common bile duct (CBD) is identified in situ, removed, and placed into a solution of Dulbeccos Moditied Eagles Medium (DMEM). The associated pancreatic acinar and islet issue, as well as attached blood vessels are then removed by dissection with forceps. The CBD, along with its associated branches, the main pancreatic ducts, are then sliced transversely into approximately 300 µm long micro-organ explants or individually dispersed single cells. These specimens are then cultured in DNTEM with the addition of growth factors, either in the presence or absence of collagen type 1 or matrigel, as a growth substrate. Effectiveness of the growth factors in stimulating proliferation is judged by the incorporation of bromodeoxyuridine (BrdU) into DNA by the responding cells. Antibodies to BrdU are used to visualize and characterize the short term responses (24-48 hr). The long term response is judged by the ability of these populations of cells to be grown and expanded in cell culture as a result of specific growth factor addition. Three different growth factors (EGF, TGF-alpha, and bFGF) are used to differentiate progenitor cells at concentrations 1 ng/ml, 10 ng/ml and 100 ng/ml. Activation of proliferation as assessed by BrdU labeling occurred with administration of 10 ng/ml of growth factor EGF within a span of 24 hr. There is no difference observed between 10 and 100 ng/ml dose. Addition of EGF to the CBD tissue explant results in proliferation of distinct cells and in clustering of these cells. Preliminary long term growth experiments indicate that there does exist a large proliferative potential within the CBD cadaveric tissue that can be maintained in culture for at least 21 days.

### 6.17. Progenitor cells for treating liver diseases

d-Galactosamine is a compound capable of inducing injury which is similar to the lesion of viral hepatitis of human beings, and is used to induce a model of hepatitis. Carbon tetrachloride generates free radicals with a very high reactivity by the action of drug metabolizing enzyme systems in liver cells, and these free radicals can strongly depress the cell activity by combining with protein of the liver cell membranes or can cause peroxidation of membrane lipids of the organelles, thus leading to necrosis of liver cells and accumulation of liver fats. Accordingly, these compounds are widely used as test models of acute drug-induced hepatitis of human beings, e.g., fatty liver, chronic hepatitis, and liver cirrhosis.

Therefore, in this example, the present inventors conduct tests in accordance with the method reported in detail in the U.S. Pat. No. 4,898,890, incorporated herein by reference so as to confirm the efficacy of cadaveric progenitor cells in accordance with the present invention. Wistar strain male rats each weighing 180 to 200 g are intraperitoneally injected with 250 mg per kilogram body weight of d-galactosamine dissolved in 5 ml per kilogram body weight of physiological saline solutions. The serum of the blood samples is examined by measuring glutamic-oxaloacetic transaminase (GOT), glutamic-pyruvic transaminase (GPT), and ALP by an automatic analyser. A liver injury-induced placebo control group is treated in exactly the same manner as that of the group in which about 1-5 x 10⁴-10⁷ liver progenitor cadaveric cells are administered directly into the injured liver except that rats in the placebo group are administered with a medium placebo solution in place of the suspensions of progenitor cells. In another series of experiments the livers of rats are injured with carbon tetrachloride instead. The liver injury-induced animals show the obvious increase in GOT, GPT, and ALP when compared with a non-injured control group. The rats, which are treated with progenitor cells demonstrate marked suppression of increase in GOT, GPT, and ALP, when compared with the liver-injury induced control not treated with hepatic progenitors. The results show that progenitor cells suppress or even reverse and certainly protect from d-galactosamine- and carbon tetrachloride-induced injury to the liver.

An 11-year-old girl who presented with a liver disease, hyperbilirubinemia, that causes excess amounts of bilirubin, a substance produced by the liver, to accumulate in her blood is required to spend 12 to 15 hours a day under ultraviolet lights as treatment, a process called phototherapy. After the hepatic cell transplant from a cadaver donor directly into her liver (portal vein), her bilirubin levels are noted as having declined dramatically, and now she is functioning although she still has to spend about four to six hours in phototherapy.

Thus, this application of cadaveric hepatic progenitors is useful in the prevention and therapy of liver malfunction and injury including but not limited to viral hepatitis, fatty liver, chronic hepatitis, fibrosis, and liver cirrhosis. It is also clear that the instant method allows to prevent and/or treat the liver metabolic dysfunction and/or injury caused by other causes such as chemotherapy, or drug abuse, or alcohol abuse for example. There are many drugs and substances possessing the tendency to cause liver injury and these comprise, without limitation, analgesics, antipyretics, anti-inflammatory drugs, and anti-rheumatic drugs such as acetaminophen, aspirin, phenylbutazone, sulindac, ibufenac, gold compounds, etc. Antibiotics: aminoglycosides, polypeptides, cephalosporins, penicillins, tetracyclines, etc. Chemotherapeutic agents: sulfa drugs, isoniazides, etc. Anti-cancer drugs: mitomycin C, cis-platinum, 6-MP, nitrosoureas, etc. Anesthetics: halothane, methoxyflurane, etc. Psychotropic drugs: chlorpromazines, diazepams, barbitals, etc. Diuretics: thiazides, etc.

These and other useful applications are obvious to those skilled in the art. The specific examples of foreseen liver diseases include but are not limited to Alagille syndrome, alcoholic liver disease, alpha-1-antitrypsin deficiency, autoimmune hepatitis, biliary atresia, biliary ductopenia, bone marrow failure, Budd-Chiari syndrome, Byler disease, Crigler-Najjar syndrome, Caroli disease, cholestatic pruritus, cholelithiasis, conjugated hyperbilirubinemia, chronic graft-versus-host disease, cryptogenic liver disease, diabetes, Dubin-Johnson syndrome, erythrohepatic protoporphyria, extrahepatic bile duct carcinoma, familial hypercholesterolemia, galactosemia, Gilbert syndrome, glycogen storage disease, hemangioma, hemochromatosis, hepatic encephalopathy, hepatocholangitis, hepatomalacia, hepatomegalia, hepatocarcinoma, hepatoblastoma, hereditary hemochromatosis, jaundice, intrahepatic cholestasis, liver cysts, liver transplantation, liver failure associated with Bacillus cereus, mixed cryoglobulinemia, ornithine transcarbamylase deficiency, peliosis hepatis, porphyria cutanea tarda, primary biliary cirrhosis, refractory ascites, Rotor syndrome, sarcoidosis, sclerosing cholangitis, steatosis, Summerskill syndrome, thrombocytopenia, tyrosinanemia, variceal bleeding, venocclusive disease of the liver, and Wilson disease.

### 6.18. Preparation of Progenitor Cells

This example provides steps for an isolation of committed and uncommitted liver progenitor cells. While various techniques are known in the art, one of preferred embodiments is disclosed in detail with understanding that other preparation techniques are equally suitable as long as they are agreeable with desired goals. For examples of preferred, non-limiting techniques see for example U. S. Pat. Nos. 5,807,686, 5,916,743, 5,672,346, 5,681,559, 5,665,557, 5,672,346, and 5,663,051 as incorporated herein by way of reference.

Pluripotent or committed hepatic, low density liver cells can be preliminary isolated using either Percoll or other suitable density gradients such as Histopaque and after centrifugation, washed twice with media and resuspended in 10 ml of elutriation media. For counterflow elutriation, the washed low density mononuclear cells are injected via a sampling site coupler into the inlet stream of a Beckman J6M/E centrifuge equipped with a JE-5 rotor and standard chamber. However, any of a number of commercial continuous flow centrifuges and elutriators that preferably employ disposable plastic insets including chamber means for facilitating density based separation can be used, such as the "Fenwal Models CS 3000" and "Autopheresis C" sold by Baxter International Inc, of Deerfield, IL; "IBM Model 2997" sold by Cobe manufacturing of Lakewood, CO. The choice of instruments is up to one skilled in the art. A peristaltic pump (Cole Palmer Instruments, Chicago, IL) provides continuous flow of elutriation medium, which is 0.9% normal saline solution with 100 mg/dl D-glucose, 0.3 mM disodium ethylenediaminetetraacetic acid (EDTA) and 50 mg/dl bovine serum albumin with pH adjusted to 7.2. The medium is sterilized prior to use. Cells are delivered at a total flow rate of 15 ml/min, rotor speed of 900g and at room temperature. After 100 ml of eluate are collected, the flow rate is increased to 25 ml/min. With the rotor speed held constant, the flow rates are sequentially increased to 29 ml/min, 33 ml/min, and 37 ml/min, collecting 200 ml with each increment. The cells that remain in the chamber are captured by turning the rotor off and flushing the chamber with 100 ml of elutriation media. Each cell fraction is washed and centrifuged at 300g for 10 minutes. Suitable fractions are collected, viability is determined by trypan blue dye exclusion and cell recoveries are determined with cell counter (Coulter Electronics, Hialeah, FL).

Alternatively liver cells are not processed through density gradient separation and are suspended in phosphate buffered saline (PBS), pH 7.4, containing 5% fetal calf serum, 0.01% EDTA wt/vol.; and 1.0 g/l D-glucose, and injected directly into a Beckman counterflow centrifugal elutriation system at 10°C at a rotor speed of 1,950 rpm using a JA-17 rotor and standard separation chamber (Beckman Instruments) and samples are eluted at flow rates between 12 and 14 ml/min. Thus this method is versatile and does not necessarily have to rely on density gradient separation.

The progenitor cells obtained in the suitable fractions generally have cell diameters in a range of 8.0 to 9.4 microns; the majority of the cells had diameters that fell within a range of 8.3 to 9.2 microns. These diameters are measured according to techniques known in the art. If necessary, further selection either positive or negative, based on cell markers is carried out.

A variety of other antibodies known to those of skill in the art can be used alone or in combination with liver progenitor markers supra. The choice will depend upon the cell type desired to be isolated or enriched and include, but are not limited to, antibodies specific to hematopoietic and lymphoid antigens such as, anti-CD2, anti-CD2R, anti-CD3, anti-CD4, anti-CD5 and anti-CD8 specific for T cells; anti-CD6 specific for T-cell subset and B-cell subset; anti-CD7 specific for major T-cell subset; anti-CD 12, anti-CD19 and anti-CD20, anti-CD72, anti-CDw78, specific for B cells; anti-CD13 and anti-CD14 specific for monocytes; anti-CD 16 and anti-CD56 specific for natural killer cells; anti-CD41 for platelets; anti-CD1a, CDlb and CD1c specific for cortical thymocytes and Langerhans cells; anti-CD9 specific for pre-B-cells, monocytes & platelets; anti-CD10 specific for lymphoid progenitor cells, C-All and granuloytes; anti-CD11a specific for leucocytes; anti-CD11b specific for granulocytes, monocytes and natural killer cells; anti-CD11c specific for monocytes, granulocytes, natural killer cells and hairy cell leukemia; anti-CD15 specific for granulocytes; anti-CDw17 specific for granulocytes, monocytes and platelets; anti-CD18 specific for leucocytes; anti-CD21 specific for mature B-cells; anti-CD22 specific for B-cells cytoplasm and mature B-cells; anti-CD23 specific for activated B-cells; anti-CD24 specific for B-cells and granulocytes; anti-CD25 and anti-CD26 specific for activated T- and B-cells and activated macrophages; anti-CD27 and anti-CD28 specific for major T-cell subset; anti-CD30 specific for activated T- and B-cells and Sternberg Reed cells; anti-CD31 specific for platelets, monocytes/macrophages, granulocytes and B-cells; anti-CDw32 specific for macrophages, granulocytes, B-cells and eosinophils; anti-CD33 specific for monocytes, myeloid progenitor cells and myeloid leukemias; anti-CD34 specific for hematopoietic precursor cells; anti-CD35 specific for granulocytes, monocytes, B-cells, some NK cells, and erythrocytes; anti-CD36 specific for monocytes/macrophages and platelets; anti-CD37 specific for mature B-cells; anti-CD38 specific for plasma cells, thymocytes and activated T-cells; anti-CD39 specific for mature B-cells; anti-CD40 specific for B-cells and carcinoma; anti-CD42 and 42b specific for platelets and megakaryocytes; anti-CD43 specific for leucocytes except circulating B-cells; anti-CD44 specific for leucocytes and Red cells; anti-CD45 specific for leucocytes; anti-CD45RO specific for T-cells, B-cells subset, monocytes and macrophages; anti-CD45RA specific for B-cells, monocytes and T-cell subset; anti-CD45RB specific for B-cells, T-cells subset, monocytes macrophages and granulocytes; anti-CD46, CD55, CD58 and CD59 specific for hematopoietic and non-hematopoietic cells; anti-CD47 specific for all cell types; anti-CD48 specific for leucocytes and neutrophils; anti-CDw49b specific for platelets, activated & long-term cultivated T-cells; anti-CDw49d specific for monocytes, T-cells & B-cells; anti-CDw49f specific for platelets and megakaryocytes; anti-CDw50 & CDw52 specific for leucocytes; anti-CD51 specific for platelets; anti-CD53 specific for leucocytes including normal and neoplastic plasma cells; anti-CD54 specific for endothelial cells; anti-CDw60 specific for T-cells subset and platelets; anti-CD61 specific for platelets & megakaryocytes; anti-CD62 specific for activated platelets; anti-CD63 specific for activated platelets, monocytes/macrophages; anti-CD64 specific for monocytes (upregulated interferon gamma.); anti-CDw65 specific for granulocytes and heterogenous reactivity with monocytes; anti-CD66 & 67 specific for granulocytes; anti-CD68 specific for monocytes and macrophages; anti-CD69 specific for activated B- and T-cells, activated macrophages, and natural killer cells; anti-CDw70 specific for activated T- and B-cells, Sternberg-Reed cells, and anaplastic large cell lymphoma; anti-CD71 specific for activated T- and B-cells, macrophages, proliferating cells; anti-CD73 specific for B-cell subset and T-cell subset; anti-CD74 specific for B-cells and monocytes/macrophages; anti-CDw75 specific for mature B-cells; anti-CD76 specific for mature B-cells and T-cell subset; anti-CD77 specific for follicular center B-cells; antibodies to cytokines and growth factors (e.g. IL1-IL13, EGF, IGF I and II, TGF-alpha and beta, TNF-alpha and beta, FGF, NGF, CIF, IFN-alpha and beta, CSF's); viral antigens (e.g. Hepatitis B virus envelope proteins or HIV envelope proteins), hormones, cellular or tumor associated antigens or markers, adhesion molecules, hemostasis molecules, and endothelial cells. Other markers and enrichment procedures known in the art are equally suitable such as disclosed for example in U.S. Pat. No. 5,840,502 incorporated by reference.

All of the above-cited references and publications are each hereby incorporated by reference in its respective entirety.

While preferred embodiments of the invention have been illustrated and described, it will be appreciated that various changes can be made therein without departing from the spirit and scope of the invention. Those skilled in the art will recognize, or be able to ascertain using no more than routine experimentation, many equivalents to the specific embodiments of the invention described herein. Such equivalents are intended to be encompassed by the following claims

## Claims

1. A method of processing non-fetal donor tissue to obtain an enriched population of progenitor cells comprising:
(a) providing non-fetal donor tissue that would be considered unsuitable for an organ transplantation; and
(b) processing said non-fetal donor tissue to obtain an enriched population of progenitor cells.

2. The method of claim 1 in which the non-fetal donor tissue, which would be considered unsuitable for an organ transplantation, is obtained from a donor whose heartbeat has ceased.

3. The method of claim 2 in which the donor tissue is obtained within about one, three or six hours after the heartbeat ceased.

4. The method of claim 1 or 2 in which the donor tissue is cooled.

5. The method of claim 4 in which the donor tissue is cooled to about 4°C.

6. The method of claims 1-5 in which the donor is a neonate, an infant, a child, a juvenile, or an adult.

7. The method of claim 1-6 in which the donor is a pig or a primate.

8. The method of claim 1-7 in which the donor tissue is selected from the group consisting of adrenal gland, blood vessel, bone marrow, cornea, retina, islets of Langerhans, bile duct, lens, liver, lung, kidney, heart, gut, ovary, pancreas, prostate, parathyroid, pineal, pituitary, skin, testis, bladder, brain, spinal cord, spleen, thymus, or thyroid.

9. The method of claims 1-8 in which the processing step provides a substantially single cell suspension or an explant.

10. The method of claim 9 in which the processing step additionally comprises selecting from the suspension those cells that express at least one marker associated positively or negatively with at least one progenitor cell lineage.

11. The method of claim 9 in which the processing step additionally comprises a debulking step, to provide a debulked cell suspension enriched in progenitors exhibiting at least one marker associated with at least one progenitor cell lineage.

12. The method of claim 9 in which the at least one progenitor cell lineage includes at least one of hepatic, hematoietic, stromal, or mesenchymal cell lineage.

13. A method of procuring liver progenitor cells, comprising:
(a) providing a non-beating heart donor as a liver tissue source; and
(b) processing the liver tissue to obtain the progenitor cells.

14. the method of claim 13 in which the donor is a mammal.

15. The method of claim 14 in which the mammal is a human.

16. The method of claims 13-15 in which the progenitor cells have the capacity to develop into hepatocytes, biliary cells, or a combination thereof.

17. The method of claims 13-16 in which the cells of the donor express at least one of alpha-fetoprotein, albumin, bone sialoprotein, CD 14, CD34, CD38, CD90, CD45, CD117, ICAM-1, collagen type I, collagen type II, collagen type III, glycophorin A, or osteopontin.

18. A method of providing a tissue having at least one progenitor cell population as a source of progenitor cells, comprising:
(a) providing a donor having a non-beating heart;
(b) harvesting the tissue from the donor, the tissue having at least one progenitor cell population; and
(c) processing further the harvested tissue to obtain progenitor cells.

19. A method of processing fetal human tissue to obtain an enriched population of human liver progenitor cells comprising:
(a) providing fetal human tissue that would be considered unsuitable for a cell or an organ transplantation; and
(b) processing said fetal human tissue to obtain an enriched population of liver progenitor cells.

20. A method of providing a tissue having at least one diploid cell population as a source of diploid cells, comprising:
(a) harvesting a tissue from a donor having a non-beating heart at a time when the tissue is harvested, the tissue harvested being suspected of having at least one diploid cell population;
(b) processing the harvested tissue to obtain a population of cells substantially enriched in diploid cells.

21. The method of claim 20 in which the donor is not a fetus.

22. The method of claim 21 in which the donor is a neonate, an infant, a child, a juvenile, or an adult.

23. The method of claims 20-22 in which the diploid cells include progenitors.

24. The method of claims 20-23 in which the processing step comprises processing the harvested tissue to provide a substantially single cell suspension.

25. The method of claim 24 in which the processing step further comprises separating the substantially single cell suspension into two or more fractions.

26. The method of claim 25 in which the separating step separates larger cells from smaller cells, higher density cells from lower density cells, or both.

27. The method of claim 26 in which one or more fractions consisting essentially of smaller cells, lower density cells, or both, are further processed to provide a population of cells substantially enriched in diploid cells.

28. The method of claim 23 in which the diploid cells include progenitors that express alpha-fetoprotein.

29. The method of claim 28 in which the progenitors include liver progenitors.

30. The method of claims 20-29 in which the tissue is harvested within about one, two, three or six hours after the heartbeat ceased.

31. The method of claims 20-30 in which the tissue is selected from the group consisting of adrenal gland, blood vessel, bone marrow, cornea, retina, islets of Langerhans, bile duct, lens, liver, lung, kidney, heart, gut, ovary, pancreas, prostate, parathyroid, pineal, pituitary, skin, testis, bladder, brain, spinal cord, spleen, thymus, or thyroid.

32. A composition comprising a population of cells substantially enriched in diploid cells obtained by the method of claims 20-31.

33. The composition of claim 32 in which the diploid cells include progenitors that express alpha-fetoprotein.
